# EUROPEAN PATENT SPECIFICATION

(11) **EP 2 421 981 B1**
(45) Date of publication and mention of the grant of the patent: **14.08.2013**
(21) Application number: 10767702.3
(22) Date of filing: 21.04.2010
(51) Int. Cl.: C12N 15/85, C12P 21/00, A61K 35/44

(54) **CELL LINES THAT PRODUCE PROSTAGLANDIN F2 ALPHA (PGF2A) AND USES THEREOF**
ZELLLINIEN ZUR PRODUKTION VON PROSTAGLANDIN-F2-ALPHA (PGF2A) UND VERWENDUNGEN DAVON
LIGNÉES CELLULAIRES QUI PRODUISENT DE LA PROSTAGLANDINE F2 ALPHA (PGF2A) ET LEURS UTILISATIONS

(30) Priority: 23.04.2009 US 171921 P
(43) Date of publication of application: 29.02.2012
(73) Proprietor: Neurotech USA, Inc., Cumberland, RI 02864 (US)
(72) Inventor: TAO, Weng, Lincoln,RI 02865 (US); KAUPER, Konrad, Sutton,MA 01590 (US); STABILA, Paul, Coventry,RI 02816 (US); LING, Vincent, Walpole,MA 02081 (US)
(74) Representative: Mintz Levin Cohn Ferris Glovsky and Popeo LLP
(86) International application number: PCT/US2010/031899
(87) International publication number: WO 2010/123994

(56) References cited:
- WO-A1-2007/127965
- US-A1- 2003 082 141
- US-A1- 2006 292 690
- US-A1- 2007 071 734
- DATABASE Geneseq [Online] 5 May 2005 (2005-05-05), "Human prostaglandin-endoperoxide synthase 2 (COX-2) gene.", XP002671439, retrieved from EBI accession no. GSN:ADX85144 Database accession no. ADX85144
- VICHAI V ET AL: "Positive feedback regulation of COX-2 expression by prostaglandin metabolites", INFLAMMATION RESEARCH, vol. 54, no. 4, April 2005 (2005-04), pages 163-172, XP002671440, ISSN: 1023-3830
- DEBEY SVENJA ET AL: "Regulation of cyclooxygenase-2 expression by iloprost in human vascular smooth muscle cells. Role of transcription factors CREB and ICER.", BIOCHEMICAL PHARMACOLOGY, vol. 65, no. 6, 15 March 2003 (2003-03-15) , pages 979-988, XP002671441, ISSN: 0006-2952
- BOROVJAGIN ANTON V ET AL: "Complex mosaicism is a novel approach to infectivity enhancement of adenovirus type 5-based vectors", CANCER GENE THERAPY, vol. 12, no. 5, May 2005 (2005-05), pages 475-486, XP002671442, ISSN: 0929-1903
- YEOM ET AL.: 'RraA rescues Escherichia coli cells over-producing RNase E from growth arrest by modulating the ribonucleolytic activity' BIOCHEMICAL AND BIOPHYSICAL RESEARCH COMMUNICATIONS vol. 345, 2006, pages 1372 - 1376

## Description

### RELATED APPLICATIONS

This application claims priority to USSN 61/171,921, filed April 23, 2009, which is herein incorporated by reference in its entirety.

### FIELD OF THE INVENTION

The present invention relates generally to the field of encapsulated cell therapy.

### BACKGROUND OF THE INVENTION

Many clinical conditions, deficiencies, and disease states can be remedied or alleviated by supplying to the patient one or more biologically active molecules produced by living cells or by removing from the patient deleterious factors which are metabolized by living cells. In many cases, these molecules can restore or compensate for the impairment or loss of organ or tissue function. Accordingly, many investigators have attempted to reconstitute organ or tissue function by transplanting whole organs, organ tissue, and/or cells, which provide secreted products or affect metabolic functions. However, while such transplantation can provide dramatic benefits, it is limited in its application by the relatively small number of organs that are suitable and available for grafting. Moreover, in general, transplantation patients must be immunosuppressed in order to avert immunological rejection of the transplant, which results in loss of transplant function and eventual necrosis of the transplanted tissue or cells. Likewise, in many cases, the transplant must remain functional for a long period of time, even for the remainder of the patient's lifetime. It is both undesirable and expensive to maintain a patient in an immunosuppressed state for a substantial period of time.

A number of vision-threatening disorders of the eye exist for which additional good therapies are still needed. One major problem in treatment of such diseases is the inability to deliver therapeutic agents into the eye and to maintain them there at therapeutically effective concentrations.

Many growth factors have shown promise in the treatment of ocular disease. For example, BDNF and CNTF have been shown to slow degeneration of retinal ganglion cells and decrease degeneration of photoreceptors in various animal models. *See, e.g.,* Genetic Technology News, vol. 13, no. 1 (Jan. 1993). Additionally, nerve growth factor has been shown to enhance retinal ganglion cell survival after optic nerve section and has also been shown to promote recovery of retinal neurons after ischemia. *See, e.g.,* Siliprandi, et al., Invest. Ophthalmol. & Vis. Sci., 34, pp. 3232-3245 (1993). In addition, CNTF has been successfully delivered to the human eye using encapsulated cells. (*See* Sieving et al., PNAS 103(10):3896-901 (2006) (incorporated herein by reference)).

A desirable alternative to transplantation procedures is the implantation of cells or tissues within a physical barrier which will allow diffusion of nutrients, metabolites, and secreted products, but will block the cellular and molecular effectors of immunological rejection. A variety of devices which protect tissues or cells producing a selected product from the immune system have been explored. *See, e.g.,* US Patent No. 5,158,881; WO92/03327; WO91/00119; and WO93/00128, each of which is incorporated herein by reference in its entirety. These devices include, for example, extravascular diffusion chambers, intravascular diffusion chambers, intravascular ultrafiltration chambers, and implantation of microencapsulated cells. *See* Scharp, D. W., et al., World J. Surg., 8, pp. 221-9 (1984). *See, e.g.,* Lim et al., Science 210: 908-910 (1980); Sun, A. M., Methods in Enzymology 137: 575-579 (1988); WO 93/03901; and U.S. Pat. No. 5,002,661. The use of such devices would alleviate the need to maintain the patient in an immunosuppressed state. However, none of these approaches have been satisfactory for providing long-term transplant function.

Thus, methods of delivering appropriate quantities of needed substances, such as, for example, neurotrophic factors, anti-angiogenic factors, anti-inflammatory factors, enzymes, hormones and/or other factors, or of providing other needed metabolic functions, to the eye for an extended period of time are needed.

### SUMMARY OF THE INVENTION

The invention provides a cell line for use in treating ophthalmic disorders, wherein said cell line comprises an ARPE-19 cell genetically engineered to express the human Cox-2 (hCox-2) enzyme encoded by the nucleic acid sequence of SEQ ID NO: 1; and wherein said cell-line produces PGF2a in therapeutically effective quantities and is for implantation into the eye of a patient.

A nucleic acid molecule of disclosed herein (*e.g*., a nucleic acid molecule having the nucleotide sequence of SEQ ID NO:1, encoding a polypeptide having the sequence of SEQ ID NO:2, or a complement of any of these nucleotide sequences) can be isolated using standard molecular biology techniques and the sequence information provided herein. Using all or a portion of these nucleic acid sequences a hybridization probe, polypeptides can be isolated using standard hybridization and cloning techniques (*e.*g., as described in Sambrook et al., (eds.), MOLECULAR CLONING: A LABORATORY MANUAL 2nd Ed., Cold Spring Harbor Laboratory Press, Cold Spring Harbor, NY, 1989; and Ausubel, et al., (eds.), CURRENT PROTOCOLS IN MOLECULAR BIOLOGY, John Wiley & Sons, New York, NY, 1993.)

Any of the nucleic acids described herein can be amplified using cDNA, mRNA or, alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to hCox-2 nucleotide sequences can be prepared by standard synthetic techniques, *e.g*., using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment, an oligonucleotide comprising a nucleic acid molecule less than 100 nt in length would further comprise at least 6 contiguous nucleotides of SEQ ID NO:1 or a complement thereof. Oligonucleotides may be chemically synthesized and may be used as probes.

A further disclosure is an isolated nucleic acid molecule comprises a nucleic acid molecule that is a complement of the nucleotide sequence shown in SEQ ID NO:1. A nucleic acid molecule that is complementary to these nucleotide sequences is one that is sufficiently complementary to the nucleotide sequence that it can hydrogen bond with little or no mismatches, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, Van der Waals, hydrophobic interactions, etc. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Moreover, the nucleic acid molecule can comprise only a portion of the nucleic acid sequence of SEQ ID NO:1, *e.g*., a fragment that can be used as a probe or primer or a fragment encoding a biologically active portion of the hCox-2 enzyme. Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are derived from different species.

Derivatives and analogs may be full length or other than full length, if the derivative or analog contains a modified nucleic acid or amino acid. Derivatives or analogs of nucleic acids or proteins of the invention include, but are not limited to, molecules comprising regions that are substantially homologous to the nucleic acids or proteins of the invention, in various embodiments, by at least about 30%, 50%, 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e.g*. Ausubel, et al., CURRENT PROTOCOLS IN MOLECULAR BIOLOGY, John Wiley & Sons, New York, NY, 1993, and below.

Disclosed are nucleic acid molecules that differ from the nucleotide sequence shown in SEQ ID NO:1 due to degeneracy of the genetic code and thus encode the same hCox-2 enzymes as that encoded by the nucleotide sequence shown in SEQ ID NO:1.

Further disclosed is an isolated nucleic acid molecule is at least 6 nucleotides in length and hybridizes under stringent conditions to the nucleic acid molecule comprising the nucleotide sequence of SEQ ID NO:I. In another embodiment, the nucleic acid is at least 10, 25, 50, 100, 250, 500, 1000, 1500, 2000, or more nucleotides in length. In another embodiment, an isolated nucleic acid molecule hybridizes to the coding region, for example of SEQ ID NO:1.

As used herein, the term "hybridizes under stringent conditions" is intended to describe conditions for hybridization and washing under which nucleotide sequences at least 60% homologous to each other typically remain hybridized to each other. Moreover, as used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e.g*., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in Ausubel et al., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well-known in the art. See, *e.g*., Ausubel et al. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g*., as employed for cross-species hybridizations). See, *e.g*., Ausubel et al. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981, Proc Natl Acad Sci USA 78: 6789-6792.

Further disclosed is an isolated polypeptide that is at least 80% identical to a polypeptide having an amino acid sequence of SEQ ID NO:2. Alternatively, the isolated polypeptide is at least 80% homologous to a fragment (*i.e*., at least 6 contiguous amino acids) of a polypeptide having an amino acid sequence of SEQ ID NO:2. Morcover, the disclosure also includes isolated polypeptides that are at least 80% homologous to a derivative, analog, or homolog of a polypeptide having an amino acid sequence of SEQ ID NO:2. Similarly, the disclosure also provides an isolated polypeptide that is at least 80% identical to a naturally occurring allelic variant of a polypeptide having an amino acid sequence of SEQ ID NO:2. Those skilled in the art will recognize that such polypeptides should be encoded by a nucleic acid molecule capable of hybridizing to a nucleic acid molecule of SEQ ID NO:1 under stringent conditions.

As used herein, the terms "protein" and "polypeptide" are intended to be interchangeable. The invention also includes mutant or variant hCox-2 enzymes any of whose residues may be changed from the corresponding residue shown in SEQ ID NO:2, while still encoding a polypeptide that maintains its PGF2a-upregulating activities and physiological functions, or a functional fragment thereof. In the mutant or variant protein, up to 20% or more of the residues may be so changed.

In general, a hCox-2 enzyme variant that preserves PGF2a-upregulating function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution.

An "isolated" or "purified" polypeptide or biologically active portion thereof is substantially free of cellular material or other contaminating proteins or polypeptides from the cell or tissue source from which the polypeptide is derived, or substantially free from chemical precursors or other chemicals when chemically synthesized. The language "substantially free of cellular material" includes preparations of polypeptides in which the polypeptide is separated from cellular components of the cells from which it is isolated or recombinantly produced. For example, the language "substantially free of cellular material" includes preparations of hCox-2 enzyme having less than about 30% (by dry weight) of non-hCox-2 enzyme (also referred to herein as a "contaminating protein"), more preferably less than about 20% of non-hCox-2 enzyme, still more preferably less than about 10% of non-hCox-2 enzyme, and most preferably less than about 5% non-hCox-2 enzyme. When the polypeptide or biologically active portion thereof is recombinantly produced, it is also preferably substantially free of culture medium, i.e., culture medium represents less than about 20%, more preferably less than about 10%, and most preferably less than about 5% of the volume of the protein preparation.

Similarly, the language "substantially free of chemical precursors or other chemicals" includes preparations of polypeptide in which the polypeptide is separated from chemical precursors or other chemicals that are involved in the synthesis of the polypeptide. For example, the language "substantially free of chemical precursors or other chemicals" includes preparations of hCox-2 enzyme having less than about 30% (by dry weight) of chemical precursors or non-hCox-2 enzyme chemical, more preferably less than about 20% chemical precursors or non-hCox-2 enzyme chemicals, still more preferably less than about 10% chemical precursors or non-hCox-2 enzyme chemicals, and most preferably less than about 5% chemical precursors or non-hCox-2 enzyme chemicals.

Biologically active portions include peptides comprising amino acid sequences sufficiently homologous to or derived from the amino acid sequence of the hCox-2 enzyme, *e.g*., the amino acid sequence shown in SEQ ID NO:2 that include fewer amino acids than the full length polypeptides described herein, and exhibit at least one activity of the hCox-2 enzyme (*e.g*., upregulation of PGF2a production). Typitally, biologically active portions comprise a domain or motif with at least one activity of the hCox-2 enzyme.

To determine the percent homology of two amino acid sequences or of two nucleic acids, the sequences are aligned for optimal comparison purposes (*e.g*., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position *(i.e.,* as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See*, Needleman and Wunsch 1970 J Mol Biol 48: 443-453. The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e.g*., A, T, C, G, U, or I, in the case of nucleic acids) occurs in both sequences to yield the number of matched positions, dividing the number of matched positions by the total number of positions in the region of comparison (*i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

A chimeric or fusion protein can be produced by standard recombinant DNA techniques. For example, DNA fragments coding for the different polypeptide sequences are ligated together in-frame in accordance with conventional techniques, *e.g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. A fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (see, for example, Ausubel et al. (eds.) CURRENT PROTOCOLS IN MOLECULAR BIOLOGY, John Wiley & Sons, 1992). Moreover, many expression vectors are commercially available that already encode a fusion moiety (*e.g*., a GST polypeptide).

The disclosure further provides vectors containing any of the nucleic acid molecules of the invention. Specifically, this also pertains to vectors, preferably expression vectors, containing a nucleic acid encoding the polypeptides of the invention, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors

(*e.g*., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g*., replication defective retroviruses, adenoviruses and adeno-associated viruses), which serve equivalent functions.

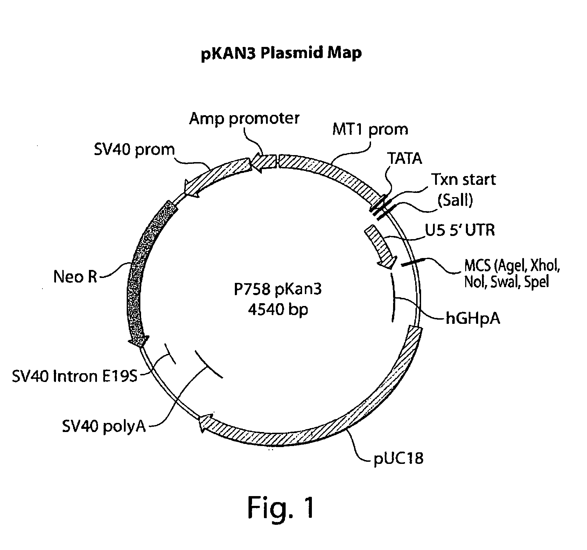

One non-limiting example of a preferred expression vector is the pKAN3 vector (*see* Figure 1).

The recombinant expression vectors of the invention can comprise any of the nucleic acids of the invention in a form suitable for expression of the nucleic acid in a host cell, which means that the recombinant expression vectors include one or more regulatory sequences, selected on the basis of the host cells to be used for expression, that is operatively linked to the nucleic acid sequence to be expressed. Within a recombinant expression vector, "operably linked" is intended to mean that the nucleotide sequence of interest is linked to the regulatory sequence(s) in a manner that allows for expression of the nucleotide sequence (*e.g*., in an *in vitro* transcription/translation system or in a host cell when the vector is introduced into the host cell).

As used herein, the term "regulatory sequence" is intended to include promoters, enhancers and other expression control elements (*e.g*., polyadenylation signals). Such regulatory sequences are described, for example, in Goeddel; GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e.g*., hCox-2 enzyme, mutant forms of hCox-2 enzyme, fusion proteins, etc.).

The recombinant expression vectors of the invention can be designed for expression of the hCox-2 enzyme in prokaryotic or eukaryotic cells. Other suitable expression systems for both prokaryotic and eukaryotic cells are known in the art. (*See*, *e.g*., Chapters 16 and 17 of Sambrook et al., MOLECULAR CLONING: A LABORATORY MANUAL. 2nd ed., Cold Spring Harbor Laboratory, Cold Spring Harbor Laboratory Press, Cold Spring Harbor, N.Y., 1989).

In addition, the invention also provides host cells or cell lines containing such vectors (or any of the nucleic acid molecules described herein). As used herein, the terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. By way of non-limiting example, the host cell may be an ARPE-19 cell containing the pKAN3 vector. However, other suitable host cells are known to those skilled in the art. The invention also provides cell lines containing the expression vector (*i.e*., the pKAN3 vector).

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding the hCox-2 enzyme can be introduced on a separate vector. Cells stably transfected with the introduced nucleic acid can be identified by drug selection (*e.g*., cells that have incorporated the selectable marker gene will survive, while the other cells die).

A host cell of the invention, such as a prokaryotic or eukaryotic host cell in culture, can be used to produce (*i.e*., express) the hCox-2 enzyme. Accordingly, the invention further provides methods for producing the hCox-2 enzyme using the host cells of the invention. In one embodiment, the method comprises culturing the host cell of invention (into which a recombinant expression vector encoding hCox-2 enzyme (SEQ ID NO:1) has been introduced) in a suitable medium such that the hCox-2 enzyme is expressed. The expressed hCox-2 enzyme upregulates production of PGF2a by the cell. In another embodiment, the method further comprises isolating the upregulated PGF2a from the medium or the host cell.

Likewise, the invention also provides cell lines of ARPE-19 cells genetically engineered to produce the hCox-2 enzyme, wherein the hCox-2 enzyme is encoded by the nucleic acid sequence selected of SEQ ID NO: 1. Similarly, the invention also provides cell lines of ARPE-19 cells genetically engineered to produce hCox-2 enzyme comprising the amino acid sequence of SEQ ID NO:2.

The cell line of the invention can be an ARPE-19 cell that is genetically engineered to express the human Cox-2 (hCox-2) enzyme, wherein the hCox-2 enzyme is encoded by the nucleic acid sequence of SEQ ID NO:1. Those skilled in the art will recognize that the expression of the hCox-2 enzyme in turn upregulates the production of prostaglandin F2 alpha (PGF2a). For example, the cell line can produce from about 1 to about 20 ng/million cells/day of PGF2a.

The invention also provides implantable cell culture devices having a core containing any of the cell lines of the invention and/or one or more ARPE-19 cells genetically engineered to express the Cox-2 (*i.e*., hCox-2) enzyme, wherein the hCox-2 enzyme is encoded by the nucleic acid of SEQ ID NO: 1, wherein the expression of hCox-2 upregulates production of PGF2a by the one or more ARPE-19 cells and a semipermeable membrane surrounding the core, wherein the membrane permits diffusion of the upregulated PGF2a therethrough.

In another embodiment, the invention also provides implantable cell culture devices containing one or more ARPE-19 cells, wherein the expression of Cox-2 enzyme by the ARPE-19 cells is increased using any suitable method known to those in the art (*e.g*., episomal replication of plasmids; gene targeting of elements that upregulate Cox-2; mutagenesis of cell lines to upregulate Cox-2 synthesis; and/or molecular evolution of Cox-2 molecules), wherein the increased expression of hCox-2 upregulates production of prostaglandin F2 alpha (PGF2a) by the one or more ARPE-19 cells and a semipermeable membrane surrounding the core, wherein the membrane permits diffusion of PGF2a therethrough.

In some embodiments the core of the devices contains a matrix disposed within the semipermeable membrane. For example, the matrix can be made from a hydrogel (*e.g*., alginate cross-linked with a multivalent ion), from extracellular matrix components, or from a plurality of monofilaments that are twisted into a yarn or woven into a mesh or are twisted into a yarn that is in non-woven strands, and wherein the cells are distributed thereon. Any suitable filamentous cell-supporting matrix made from a biocompatible material can be used. For example, suitable biocompatible materials include, but are not limited to, acrylic, polyester, polyethylene, polypropylene polyacetonitrile, polyethylene terephthalate, nylon, polyamides, polyurethanes, polybutester, silk, cotton, chitin, carbon, and/or biocompatible metals.

In some embodiments, the devices of the invention can contain a tether anchor, for example, an anchor loop, that is adapted for anchoring the device to an ocular structure.

Any of the devices of the invention can be implanted (or are suitable for implantation) into the eye. By way of non-limiting example, one or more devices can be implanted (or are suitable for implantation) into the vitreous, the aqueous humor, the Subtenon's space, the periocular space, the posterior chamber, and/or the anterior chamber of the eye. In one preferred embodiment, the device is implanted (or is suitable for implantation) in the vitreous of the eye.

The jacket of the devices of the invention can be made of a permselective, immunoisolatory membrane or from a microporous membrane. For example, the jackets are made from an ultrafiltration membrane or a microfiltration membrane. Those skilled in the art will recognize that an ultrafiltration membrane typically has a pore size of 1-100 nm, whereas a microfiltration membrane typically has a pore size of 0.1-10 µm. In other embodiments, the jacket may be made from a non-porous membrane material (*e.g*., a hydrogel or a polyurethane).

Any suitable device shape can be used in connection with the invention. For example, the devices can be configured as a hollow fiber or a flat sheet.

In some embodiments, at least one additional biologically active molecule (from a non-cellular or a cellular source) is co-delivered from the device. Those skilled in the art will recognize that the at least on additional biologically active molecule can be produced by one or more genetically engineered ARPE-19 cells in the core.

In any of the devices of the invention, the expression of hCox-2 by the cells (or cell lines) within the core upregulates production of prostaglandin F2 alpha (PGF2a). Preferably, the devices produce from about 1 to about 20 ng per day of PGF2a.

Also provided are methods for treating ophthalmic disorders by implanting the one or more implantable cell culture devices of the invention into the eye of a patient and allowing PGF2a to be expressed in therapeutically effective quantities, thereby treating the ophthalmic disorder. For example, the therapeutically effective quantity of PGF2a production is from about 1 to about 20 ng per million cells per day. Preferably, the ophthalmic disorder is glaucoma (*e.g*., open angle glaucoma). Other ophthalmic disorders to be treated include, but are not limited to, retinopathy of prematurity, diabetic macular edema, diabetic retinopathy, age-related macular degeneration, retinitis pigmentosa, cataract formation, retinoblastoma and retinal ischemia.

Those skilled in the art will recognize that production of PGF2a decreases intraocular pressure, stabilizes intraocular pressure, or both decreases and stabilizes intraocular pressure in the patient.

The invention also provides methods of delivering PGF2a to a recipient host by implanting the implantable cell culture device of the invention into a target region of the recipient host, wherein the encapsulated one or more ARPE-19 cells secrete PGF2a at the target region. Suitable target regions include, but are not limited to, the brain, ventricle, spinal cord, the aqueous and vitreous humors of the eye, and the posterior and anterior chamber of the eye. Preferred target regions are the aqueous and vitreous humors of the eye and the posterior and anterior chamber of the eye.

Those skilled in the art will recognize that in any of the methods described herein, between 0.1 pg and 1000 µg per patient per day of the PGF2a can diffuse from the implantable cell culture devices. Preferably, the devices produce from about 1 to about 20 ng per day of PGF2a.

Finally, the invention also provides methods for making the implantable cell culture devices of the invention by genetically engineering at least one ARPE-19 cell to express the nucleic acid sequence of SEQ ID NO:1 and encapsulating the genetically modified ARPE-19 cells within a semipermeable membrane. In another method, at least one ARPE-19 cell is genetically engineered to express hCox-2 enzyme having the amino acid sequence of SEQ ID NO:2, which, in turn upregulates the production of PGF2a by the ARPE-19 cells and the genetically modified ARPE-19 cells are encapsulated within a semipermeable membrane, wherein said membrane allows the diffusion of the upregulated PGF2a therethrough.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and are not intended to be limiting.

Other features and advantages of the invention will be apparent from the following detailed description and claims.

### BRIEF DESCRIPTION OF THE DRAWINGS

**Figure 1** is a schematic showing the pKAN3 Plasmid Map.

### DETAILED DESCRIPTION OF THE INVENTION

Glaucoma is a potentially blinding disease characterized by elevated intraocular pressure ("IOP"). Topical administration of prostaglandin F2 alpha ("PGF2a") has been shown to lower the elevated intraocular pressure associated with glaucoma. However, effective PGF2a dosages have unacceptable side effects, including, but not limited to, conjunctival hyperaemia (bloodshot eyes), iridial pigmentation (iris darkening), hypertrichosis (growth and darkening of the eyelashes), anterior uveitis (iris and ciliary body inflammation) and/or cystoid macular edema.

To overcome these drawbacks, synthetic PGF2a analogues ("PGAs"), such as Xalatan, Lumigan and Travatan, were created. These PGAs retain the IOP-lowering characteristics of PGF2a. However, they do not eliminate them completely.

Typically, drugs in this class are bolus delivered topically once daily in the form of drops at a concentration of approximately 1-13 micrograms per drop. In addition to the other side effects enumerated above, the ebb and flow of drug availability associated with bolus dosing can also cause fluctuations in patient IOP, which may be a major risk factor for patients suffering from glaucoma.

Cellular PGF2a is synthesized from arachadonic acid. The enzyme cyclooxygenase (Cox) catalyzes the committed steps in the biosynthesis of PGF2a, as well as other prostaglandins. There are two isoforms of cyclooxygenase, called Cox-1 and Cox-2. Cox-1 is considered a housekeeping gene, which is constitutively expressed and is responsible for normally low, basal levels of PGF2a production. Cox-2 expression is highly regulated and can be induced by various stimuli, which, in turn, results in increased physiological levels of prostaglandin F2 alpha.

To create the cell lines of the invention, the human cDNA clone for human Cox-2 (GenBank Accession No. NM_000963.1) was subcloned into the Neurotech expression vector pKAN3 (*see* Figure 1), to create a hCox-2 expression vector referred to herein as P777. P777 was then used to stably transfect ARPE-19 cells. Several stable cell lines were found to produce high levels of PGF2a (*e.g*., 1-20 ng per million cells per day), as measured by ELISA.

Those skilled in the art will recognize that, because Cox-2 is an intracellular enzyme, immunogenicity is not a concern. Accordingly, while the compositions and methods described in detail herein utilize the human Cox-2 gene, the Cox-2 gene from any other species can be used in place of hCox-2.

The invention involves methods and compositions that are capable increasing the expression of the Cox-2 enzyme within a cell. This increased expression of Cox-2 enzyme, in turn, leads to increased production of prostaglandin F2 alpha (PGF2a). Any suitable method of increasing the level of Cox-2 expression known in the art can be employed in accordance with the present invention.

For example, a gene of interest (*i.e*., a gene that encodes human cyclooxygenase 2 (hCox-2)) can be inserted into a cloning site of a suitable expression vector by using standard techniques. The nucleic acid and amino acid sequences of the human (and other mammalian) genes encoding hCox-2 are known.

A wide variety of host/expression vector combinations may be used to express the gene encoding the growth factor, or other biologically active molecule(s) of interest. Long-term, stable *in vivo* expression is achieved using expression vectors (*i.e*., recombinant DNA molecules) in which the gene encoding hCox-2 is operatively linked to a promoter that is not subject to down regulation upon implantation *in-vivo* in a mammalian host. Accordingly, such expression vectors would typically not contain a retroviral promoter. Suitable promoters include, for example, the early and late promoters of SV40 or adenovirus, the mouse metallothionein promoter, and other known non-retroviral promoters capable of controlling gene expression.

The expression vector containing the gene of interest may then be used to transfect the desired cell line. Standard transfection techniques such as calcium phosphate co-precipitation, DEAE-dextran transfection or electroporation may be utilized. Commercially available mammalian transfection kits, such as Fugene6 (Roche Applied Sciences), may be used. Human mammalian cells can also be used. In all cases, it is important that the cells or tissue contained in the device are not contaminated or adulterated. Preferred promoters used in the disclosed constructs include the SV40 promoter, the Amp promoter and the MT1 promoter, as shown in Figure 1.

Other useful expression vectors, for example, may consist of segments of chromosomal, non-chromosomal and synthetic DNA sequences, such as various known derivatives of SV40 and known bacterial plasmids, *e.g*., pUC, pBlueScript^{™} plasmids from E. coli including pBR322, pCR1, pMB9 and their derivatives. Expression vectors containing the geneticin (G418) or hygromycin drug selection genes (Southern, P. J., In Vitro, 18, p. 315 (1981), Southern, P. J. and Berg, P., J. Mol. Appl. Genet., 1, p. 327 (1982)) are also useful. These vectors can employ a variety of different enhancer/promoter regions to drive the expression of both a biologic gene of interest and/or a gene conferring resistance to selection with toxin such as G418 or hygromycin B. A variety of different mammalian promoters can be employed to direct the expression of the genes for G418 and hygromycin B and/or the biologic gene of interest. The G418 resistance gene codes for aminoglycoside phosphotransferase (APH) which enzymatically inactivates G418 (100-500 µg/µl) added to the culture medium. Only those cells expressing the APH gene will survive drug selection usually resulting in the expression of the second biologic gene as well. The hygromycin B phosphotransferase (HPH) gene codes for an enzyme which specifically modifies hygromycin toxin and inactivates it. Genes co-transfected with or contained on the same plasmid as the hygromycin B phosphotransferase gene will be preferentially expressed in the presence of hygromycin B at 50-200 µg/ml concentrations.

Examples of expression vectors that can be employed include, but are not limited to, the commercially available pRC/CMV, pRC/RSV, and pCDNA1NEO (In Vitrogen). In one preferred embodiments, the pKAN3 vector (Neurotech USA, Inc.) is used. (*See*, Figure 1).

The viral promoter regions directing the transcription of the drug selection and BAM genes of interest are replaced with one of the above promoter sequences that are not subject to the down regulation experienced by viral promoters within the CNS. For example, the GFAP promoter would be employed for the transfection of astrocytes and astrocyte cell lines, the TH promoter would be used in PC12 cells, or the MBP promoter would be used in oligodendrocytes.

In one embodiment, the pNUT expression vector, which contains the cDNA of the mutant DHFR and the entire pUC18 sequence including the polylinker, can be used. *See, e.g*., Aebischer, P., et al., Transplantation, 58, pp. 1275-1277 (1994); Baetge et al., PNAS, 83, pp. 5454-58 (1986). The pNUT expression vector can be modified such that the DHFR coding sequence is replaced by the coding sequence for G418 or hygromycin drug resistance. The SV40 promoter within the pNUT expression vector can also be replaced with any suitable constitutively expressed mammalian promoter, such as those discussed above.

Increased expression can be achieved by increasing or amplifying the copy number of the transgene encoding the desired molecule, using amplification methods well known in the art. Such amplification methods include, *e.g*., DHFR amplification (*see*, *e.g.,* Kaufman et al., U.S. Pat. No. 4,470,461) or glutamine synthetase ("GS") amplification (*see, e.g.,* U.S. Pat. No. 5,122,464, and European published application EP 338,841).

In another example, episomal replication of plasmids can be used to increase Cox-2 enzyme expression levels within a cell. Given the right combination of replication origin and replication machinery, expression vectors can be generated that stably transcribe and produce protein while existing in an episomal form. One example is the Ori-P and EBNA system, where the Epstein-Barr nuclear antigen stimulates plasmid replication through the Ori-P locus, as in the pCEP4 expression system (In Vitrogen). In other examples, episomal retention of expression plasmids have been achieved by S/MAR replication machinery, by SV40 based systems, by BKV based systems, or by BPV based systems. Stable episomal expression systems may be sufficiently robust to permit the creation of DNA expression vector systems that affords continual Cox-2 expression that is independent on stable cell line generation.

As another example, gene targeting of elements that upregulate Cox-2 can also be used to increase Cox-2 enzyme expression levels. It is well known that promoters can be trapped by random insertion of reporter genes into the mammalian genome, and it is common practice to genetically target defined regions by inserting DNA elements into such defined regions, which affords recombination that modifies gene function. A combination of these two technologies may create a third technology where promoters and enhancers that are active for ARPE-19 cells (*i.e.,* (NTC-200 cells) can be synthesized, isolated, and reintroduced into the cells at junctions near the Cox-2 gene. For example, the targeted promoters or enhancing elements may be upstream of the Cox-2 gene and integration there will cause significant increase of Cox-2 gene synthesis. The introduced promoters may exist as single genetic element, or may exist in multiple copies or exist in a concatemer of multiple types of different expression enhancing elements. Alternatively (or additionally), enhancing regions may be targeted to non-coding regions within splice introns of Cox-2 gene, or in regions flanking the Cox-2 gene.

Those skilled in the art will also recognize that mutagenesis of cell lines can be used to upregulate Cox-2 synthesis. For example, mutagenic methods that upregulate gene synthesis, typically on a random basis, can be used. Such mutagenic methods may include, by way of non-limiting example, random integration of DNA elements and/or the application of mutagenic chemical compounds that induce DNA lesions or removes imprinting methylation for gene silencing. Those skilled in the art will recognize that progeny of cell lines subjected to such treatment(s) will, on occasion, upregulate discrete production of certain proteins. This upregulation can be detected at the transcriptional level using gene arrays, or next generation deep sequencing strategies. A parental ARPE-19 cell line (*i.e*., NTC-200) may be exposed to mutagenic procedure(s) that, in turn, may upregulate PGF2a by affecting Cox-2 synthesis. Those skilled in the art will recognize that such procedure(s) can be used without having to introduce exogenous genetic elements that encode Cox-2.

Finally, molecular evolution of Cox-2 molecules can also be used. Specifically, using current methods of molecular evolution, protein derivatives of Cox-2 may be created that have similar activities, but deviate in structure compared to Cox-2. Examples of this technique include, but are not limited to, the successful molecular evolution of fibronectin domains, immunoglobulin V-domain scaffolds, darpins, and/or lipocalins into antibody-like structures having binding functions that are not associated with the parent molecule.

With this technique, the human Cox-2 molecule may be used as a template to initiate mutagesis procedures. Alternatively (or additionally), the initiating template may be a non-related protein that have some structural similarities with Cox-2 that may allow molecular evolution to generate and enzymatic molecule. Those skilled in the art will recognize that structural similarities may be defined by, but are not limited to, Pfam domain analysis, 3-D crystal structure, and/or phylogenetic analysis.

In another embodiment, genetic templates having Cox-2 like proteins features may be identified by genetic information analysis of species other than homo sapiens. Starting with such initial templates, serial synthetic mutagenesis may be applied to create new bioactive molecules. These mutagenic procedures may include, but are not limited to, error-prone PCR, domain shuffling of a set of Cox-2 like sequence fragments, *in vivo* mutagenesis based on chemical adducts, targeted mutagenic oligonucleotide incorporation in strand synthesis, or codon-based substitutions. A successful outcome would be a Cox-2 mutein that stimulates PGF2a production in a mechanism analogous to that of Cox-2. The subsequent library of molecules may contain full-length Cox-2 derived gene products, or shortened or truncated gene products which code for a Cox-2 mutein. Derived Cox-2 muteins can be defined by screening in bioassays that measuring upregulation of PGF2a release from transfected cells.

The gene encoding the hCox-2 enzyme has been cloned and its nucleotide sequences published. (GenBank Accession NM_000963.1). This gene is publicly available from depositories such as the American Type Culture Collection (ATCC) or various commercial sources. Alternatively, genes encoding the biologically active molecules useful in this invention that are not publicly available may be obtained using standard recombinant DNA methods such as PCR amplification, genomic and cDNA library screening with oligonucleotide probes.

The nucleotide and polypeptide sequences for hCox-2 are shown below, as SEQ ID NOS: 1 and 2, respectively.

1 caattgtcat acgacttgca gtgagcgtca ggagcacgtc caggaactcc tcagcagcgc
61 ctccttcagc tccacagcca gacgccctca gacagcaaag cctacccccg cgccgcgccc
121 tgcccgccgc tcggatgctc gcccgcgccc tgctgctgtg cgcggtcctg gcgctcagcc
181 atacagcaaa tccttgctgt tcccacccat gtcaaaaccg aggtgtatgt atgagtgtgg
241 gatttgacca gtataagtgc gattgtaccc ggacaggatt ctatggagaa aactgctcaa
301 caccggaatt tttgacaaga ataaaattat ttctgaaacc cactccaaac acagtgcact
361 acatacttac ccacttcaag ggattttgga acgttgtgaa taacattccc ttccttcgaa
421 atgcaattat gagttatgtc ttgacatcca gatcacattt gattgacagt ccaccaactt
481 acaatgctga ctatggctac aaaagctggg aagccttctc taacctctcc tattatacta
541 gagcccttcc tcctgtgcct gatgattgcc cgactccctt gggtgtcaaa ggtaaaaagc
601 agcttcctga ttcaaatgag attgtggaaa aattgcttct aagaagaaag ttcatccctg
661 atccccaggg ctcaaacatg atgtttgcat tctttgccca gcacttcacg catcagtttt
721 tcaagacaga tcataagcga gggccagctt tcaccaacgg gctgggccat ggggtggact
781 taaatcatat ttacggtgaa actctggcta gacagcgtaa actgcgcctt ttcaaggatg
841 gaaaaatgaa atatcagata attgatggag agatgtatcc tcccacagtc aaagatactc
901 aggcagagat gatctaccct cctcaagtcc ctgagcatct acggtttgct gtggggcagg
961 aggtctttgg tctggtgcct ggtctgatga tgtatgccac aatctggctg cgggaacaca
1021 acagagtatg cgatgtgctt aaacaggagc atcctgaatg gggtgatgag cagttgttcc
1081 agacaagcag gctaatactg ataggagaga ctattaagat tgtgattgaa gattatgtgc
1141 aacacttgag tggctatcac ttcaaactga aatttgaccc agaactactt ttcaacaaac
1201 aattccagta ccaaaatcgt attgctgctg aatttaacac cctctatcac tggcatcccc
1261 ttctgcctga cacctttcaa attcatgacc agaaatacaa ctatcaacag tttatctaca
1321 acaactctat attgctggaa catggaatta cccagtttgt tgaatcattc accaggcaaa
1381 ttgctggcag ggttgctggt ggtaggaatg ttccacccgc agtacagaaa gtatcacagg
1441 cttccattga ccagagcagg cagatgaaat accagtcttt taatgagtac cgcaaacgct
1501 ttatgctgaa gccctatgaa tcatttgaag aacttacagg agaaaaggaa atgtctgcag
1561 agttggaagc actctatggt gacatcgatg ctgtggagct gtatcctgcc cttctggtag
1621 aaaagcctcg gccagatgcc atctttggtg aaaccatggt agaagttgga gcaccattct
1681 ccttgaaagg acttatgggt aatgttatat gttctcctgc ctactggaag ccaagcactt
1741 ttggtggaga agtgggtttt caaatcatca acactgcctc aattcagtct ctcatctgca
1801 ataacgtgaa gggctgtccc tttacttcat tcagtgttcc agatccagag ctcattaaaa
1861 cagtcaccat caatgcaagt tcttcccgct ccggactaga tgatatcaat cccacagtac
1921 tactaaaaga acgttcgact gaactgtaga agtctaatga tcatatttat ttatttatat
1981 gaaccatgtc tattaattta attatttaat aatatttata ttaaactcct tatgttactt
2041 aacatcttct gtaacagaag tcagtactcc tgttgcggag aaaggagtca tacttgtgaa
2101 gacttttatg tcactactct aaagattttg ctgttgctgt taagtttgga aaacagtttt

2161 tattctgttt tataaaccag agagaaatga gttttgacgt ctttttactt gaatttcaac
2221 ttatattata agaacgaaag taaagatgtt tgaatactta aacactatca caagatggca
2281 aaatgctgaa agtttttaca ctgtcgatgt ttccaatgca tcttccatga tgcattagaa
2341 gtaactaatg tttgaaattt taaagtactt ttggttattt ttctgtcatc aaacaaaaac
2401 aggtatcagt gcattattaa atgaatattt aaattagaca ttaccagtaa tttcatgtct
2461 actttttaaa atcagcaatg aaacaataat ttgaaatttc taaattcata gggtagaatc
2521 acctgtaaaa gcttgtttga tttcttaaag ttattaaact tgtacatata ccaaaaagaa
2581 gctgtcttgg atttaaatct gtaaaatcag atgaaatttt actacaattg cttgttaaaa
2641 tattttataa gtgatgttcc tttttcacca agagtataaa cctttttagt gtgactgtta
2701 aaacttcctt ttaaatcaaa atgccaaatt tattaaggtg gtggagccac tgcagtgtta
2761 tctcaaaata agaatatttt gttgagatat tccagaattt gtttatatgg ctggtaacat
2821 gtaaaatcta tatcagcaaa agggtctacc tttaaaataa gcaataacaa agaagaaaac
2881 caaattattg ttcaaattta ggtttaaact tttgaagcaa actttttttt atccttgtgc
2941 actgcaggcc tggtactcag attttgctat gaggttaatg aagtaccaag ctgtgcttga
3001 ataacgatat gttttctcag attttctgtt gtacagttta atttagcagt ccatatcaca
3061 ttgcaaaagt agcaatgacc tcataaaata cctcttcaaa atgcttaaat tcatttcaca
3121 cattaatttt atctcagtct tgaagccaat tcagtaggtg cattggaatc aagcctggct
3181 acctgcatgc tgttcctttt cttttcttct tttagccatt ttgctaagag acacagtctt
3241 ctcatcactt cgtttctcct attttgtttt actagtttta agatcagagt tcactttctt
3301 tggactctgc ctatattttc ttacctgaac ttttgcaagt tttcaggtaa acctcagctc
3361 aggactgcta tttagctcct cttaagaaga ttaaaagaga aaaaaaaagg cccttttaaa
3421 aatagtatac acttatttta agtgaaaagc agagaatttt atttatagct aattttagct
3481 atctgtaacc aagatggatg caaagaggct agtgcctcag agagaactgt acggggtttg
3541 tgactggaaa aagttacgtt cccattctaa ttaatgccct ttcttattta aaaacaaaac
3601 caaatgatat ctaagtagtt ctcagcaata ataataatga cgataatact tcttttccac
3661 atctcattgt cactgacatt taatggtact gtatattact taatttattg aagattatta
3721 tttatgtctt attaggacac tatggttata aactgtgttt aagcctacaa tcattgattt
3781 ttttttgtta tgtcacaatc agtatatttt ctttggggtt acctctctga atattatgta
3841 aacaatccaa agaaatgatt gtattaagat ttgtgaataa atttttagaa atctgattgg
3901 catattgaga tatttaaggt tgaatgtttg tccttaggat aggcctatgt gctagcccac
3961 aaagaatatt gtctcattag cctgaatgtg ccataagact gaccttttaa aatgttttga
4021 gggatctgtg gatgcttcgt taatttgttc agccacaatt tattgagaaa atattctgtg
4081 tcaagcactg tgggttttaa tatttttaaa tcaaacgctg attacagata atagtattta
4141 tataaataat tgaaaaaaat tttcttttgg gaagagggag aaaatgaaat aaatatcatt
4201 aaagataact caggagaatc ttctttacaa ttttacgttt agaatgttta aggttaagaa
4261 agaaatagtc aatatgcttg tataaaacac tgttcactgt tttttttaaa aaaaaaactt
4321 gatttgttat taacattgat ctgctgacaa aacctgggaa tttgggttgt gtatgcgaat
4381 gtttcagtgc ctcagacaaa tgtgtattta acttatgtaa aagataagtc tggaaataaa
4441 tgtctgttta tttttgtact attta (SEQ ID NO:1)

MLARALLLCAVLALSHTANPCCSHPCQNRGVCMSVGFDQYKCDCTRTGFYGENCSTPEFLTRIKLFLKPT
PNTVHYILTHFKGFWNVVNNIPFLRNAIMSYVLTSRSHLIDSPPTYNADYGYKSWEAFSNLSYYTRALPP
VPDDCPTPLGVKGKKQLPDSNEIVEKLLLRRKFIPDPQGSNMMFAFFAQHFTHQFFKTDHKRGPAFTNGL
GHGVDLNHIYGETLARQRKLRLFKDGKMKYQIIDGEMYPPTVKDTQAEMIYPPQVPEHLRFAVGQEVFGL
VPGLMMYATIWLREHNRVCDVLKQEHPEWGDEQLFQTSRLILIGETIKIVIEDYVQHLSGYHFKLKFDPE
LLFNKQFQYQNRIAAEFNTLYHWHPLLPDTFQIHDQKYNYQQFIYNNSILLEHGITQFVESFTRQIAGRV
AGGRNVPPAVQKVSQASIDQSRQMKYQSFNEYRKRFMLKPYESFEELTGEKEMSAELEALYGDIDAVELY
PALLVEKPRPDAIFGETMVEVGAPFSLKGLMGNVICSPAYWKPSTFGGEVGFQIINTASIQSLICNNVKG
CPFTSFSVPDPELIKTVTINASSSRSGLDDINPTVLLKERSTEL (SEQ ID NO:2)

Those skilled in the art will recognize that because Cox-2 is an intracellular enzyme, a variety of Cox-2 like molecules can be employed in the methods and compositions described herein. By way of non-limiting example, such Cox-2 like molecules can include compounds that have molecularly evolved from a Cox-2 template; domain shuffled Cox-2 compounds that still retain Cox-2 biological activities (*e.g*., the upregulation of PGF2a production); and/or Cox-2 orthologs/paralogs from a very distant phylogenetic species (*e.g*., jellyfish). The sequences of the Cox-2 gene from other species are known to those in the art. Moreover, some mixture of these Cox-2 like molecules can also be used.

In some preferred embodiments, the cell of choice is the ARPE-19 cell line, a spontaneously arising continuous human retinal pigmented epithelial cell line. Here, the choice of cell depends upon the intended application. The encapsulated cells may be chosen for secretion of prostaglandin 2F alpha. Cells can also be employed which synthesize and secrete agonists, analogs, derivatives or fragments of the construct, which are active. Those skilled in the art will recognize that other suitable cell types may also be genetically engineered to secrete/synthesize PGF2α described herein.

The choice of cell depends upon the intended application. The encapsulated cells may be chosen for expression of hCox-2 enzyme and/or PGF2a. Cells can also be employed which synthesize and secrete agonists, analogs, derivatives or fragments of hCox-2 and/or PGF2a that active.

To be a platform cell line for an encapsulated cell based delivery system, the cell line should have as many of the following characteristics as possible: (1) the cells should be hardy under stringent conditions (the encapsulated cells should be functional in the avascular tissue cavities such as in the central nervous system or the eye, especially in the intra-ocular environment); (2) the cells should be able to be genetically modified (the desired therapeutic factors needed to be engineered into the cells); (3) the cells should have a relatively long life span (the cells should produce sufficient progenies to be banked, characterized, engineered, safety tested and clinical lot manufactured); (4) the cells should preferably be of human origin (which increases compatibility between the encapsulated cells and the host); (5) the cells should exhibit greater than 80% viability for a period of more than one month *in vivo* in device (which ensures long-term delivery); (6) the encapsulated cells should deliver an efficacious quantity of a useful biological product (which ensures effectiveness of the treatment); (7) the cells should have a low level of host immune reaction (which ensures the longevity of the graft); and (8) the cells should be nontumorigenic (to provide added safety to the host, in case of device leakage).

The ARPE-19 cell line (*see* Dunn et al., 62 Exp. Eye Res. 155-69 (1996), Dunn et al., 39 Invest. Ophthalmol. Vis. Sci. 2744-9 (1998), Finnemann et al., 94 Proc. Natl. Acad. Sci. USA 12932-7 (1997), Handa et al., 66 Exp. Eye. 411-9 (1998). Holtkamp et al., 112 Clin. Exp. Immunol. 34-43 (1998), Maidji et al., 70 J. Virol. 8402-10 (1996); United States Patent No. 6,361,771) demonstrates all of the characteristics of a successful platform cell for an encapsulated cell-based delivery system. The ARPE-19 cell line is available from the American Type Culture Collection (ATCC Number CRL-2302). ARPE-19 cells are normal retinal pigmented epithelial (RPE) cells and express the retinal pigmented epithelial cell-specific markers CRALBP and RPE-65. ARPE-19 cells form stable monolayers, which exhibit morphological and functional polarity.

When the devices of the invention are used, preferably between 10² and 10⁸ ARPE-19 cells, most preferably 5x10² ARPE-19 cells that have been genetically engineered to express hCox-2 (which, in turn, upregulates production of PGF2a), are encapsulated in each device. In one embodiment, the device contains between 200,000 and 400,000 cells. However, a micronized device containing between 10,000 and 100,000 cells is also contemplated. (*See* WO07/078922, incorporated herein by reference). Dosage may be controlled by implanting a fewer or greater number of devices, preferably between 1 and 50 devices per patient. The devices described herein are capable of delivering between about 1 ng and about 200 ng device per day of PGF2a (*in vitro*)*.*

Techniques and procedures for isolating cells or tissues which produce a selected product are known to those skilled in the art, or can be adapted from known procedures with no more than routine experimentation.

If the cells to be isolated are replicating cells or cell lines adapted to growth *in vitro,* it is particularly advantageous to generate a cell bank of these cells. A particular advantage of a cell bank is that it is a source of cells prepared from the same culture or batch of cells. That is, all cells originated from the same source of cells and have been exposed to the same conditions and stresses. Therefore, the vials can be treated as identical clones. In the transplantation context, this greatly facilitates the production of identical or replacement devices. It also allows simplified testing protocols, which assure that implanted cells are free of retroviruses and the like. It may also allow for parallel monitoring of vehicles *in vivo* and *in vitro,* thus allowing investigation of effects or factors unique to residence *in vivo.*

As used herein, the term "individual" or "recipient" or "host" refers to a human or an animal subject.

A "biologically active molecule" ("BAM") is a substance that is capable of exerting a biologically useful effect upon the body of an individual in whom a device of the present invention is implanted. For example, hCox-2 and PGF2a are examples ofBAMs.

The terms "capsule" and "device" and "vehicle" are used interchangeably herein to refer to the ECT devices of the invention.

Unless otherwise specified, the term "cells" means cells in any form, including but not limited to cells retained in tissue, cell clusters, cell lines, and individually isolated cells.

As used herein a "biocompatible capsule" or "biocompatible device" or "biocompatible vehicle" means that the capsule or device or vehicle, upon implantation in an individual, does not elicit a detrimental host response sufficient to result in the rejection of the capsule or to render it inoperable, for example through degradation.

As used herein an "immunoisolatory capsule" or "immunoisolatory device" or "immunoisolatory vehicle" means that the capsule, upon implantation into an individual, minimizes the deleterious effects of the host's immune system on the cells within its core.

As used herein "long-term, stable expression of a biologically active molecule" means the continued production of a biologically active molecule at a level sufficient to maintain its useful biological activity for periods greater than one month, preferably greater than three months and most preferably greater than six months. Implants of the devices and the contents thereof are able to retain functionality for greater than three months *in vivo* and in many cases for longer than a year.

The "semi-permeable" nature of the jacket membrane surrounding the core permits molecules produced by the cells (*e.g*., metabolites, nutrients and/or therapeutic substances) to diffuse from the device into the surrounding host eye tissue, but is sufficiently impermeable to protect the cells in the core from detrimental immunological attack by the host.

The exclusion of IgG from the core of the vehicle is not the touchstone of immunoisolation, because in most cases IgG alone is insufficient to produce cytolysis of the target cells or tissues. Thus, for immunoisolatory capsules, jacket nominal molecular weight cutoff (MWCO) values up to 1000 kD are contemplated. Preferably, the MWCO is between 50-700 kD. Most preferably, the MWCO is between 70-300 kD. *See, e.g.,* WO 92/19195.

The instant invention also relates to biocompatible, optionally immunoisolatory, devices for the delivery of PGF2a to the eye. Such devices contain a core containing living cells that produce or secrete the hCox-2 enzyme, which, in turn, upregulates PGF2a production in the cells, and a biocompatible jacket surrounding the core, wherein the jacket has a molecular weight cut off ("MWCO") that allows the diffusion of PGF2a into the eye (*e.g.,* into the vitreous) and/or to the central nervous system, including the brain, ventricle, spinal cord.

A variety of biocompatible capsules are suitable for delivery of molecules according to this invention. Useful biocompatible polymer capsules comprise (a) a core which contains a cell or cells, either suspended in a liquid medium or immobilized within a biocompatible matrix, and (b) a surrounding jacket comprising a membrane which does not contain isolated cells, which is biocompatible, and permits diffusion of the cell-produced biologically active molecule into the eye.

Many transformed cells or cell lines are advantageously isolated within a capsule having a liquid core, comprising, *e.g*., a nutrient medium, and optionally containing a source of additional factors to sustain cell viability and function. The core of the devices of the invention can function as a reservoir for growth factors (*e.g*., prolactin, or insulin-like growth factor 2), growth regulatory substances such as transforming growth factor β (TGF-β) or the retinoblastoma gene protein or nutrient-transport enhancers (*e.*g., perfluorocarbons, which can enhance the concentration of dissolved oxygen in the core). Certain of these substances are also appropriate for inclusion in liquid media.

In addition, the instant devices can also be used as a reservoir for the controlled delivery of needed drugs or biotherapeutics. In such cases, the core contains a high concentration of the selected drug or biotherapeutic (alone or in combination with cells or tissues). In addition, satellite vehicles containing substances which prepare or create a hospitable environment in the area of the body in which a device according to the invention is implanted can also be implanted into a recipient. In such instances, the devices containing immunoisolated cells are implanted in the region along with satellite vehicles releasing controlled amounts of, for example, a substance which down-modulates or inhibits an inflammatory response from the recipient (*e.g*., anti-inflammatory steroids), or a substance which stimulates the ingrowth of capillary beds (*e.g*., an angiogenic factor).

Alternatively, the core may comprise a biocompatible matrix of a hydrogel or other biocompatible material (*e.g*., extracellular matrix components) which stabilizes the position of the cells. The term "hydrogel" herein refers to a three dimensional network of cross-linked hydrophilic polymers. The network is in the form of a gel, substantially composed of water, preferably gels being greater than 90% water. Compositions which form hydrogels fall into three classes. The first class carries a net negative charge (*e.g*., alginate). The second class carries a net positive charge (*e.g*., collagen and laminin). Examples of commercially available extracellular matrix components include Matrigel^{™} and Vitrogen^{™}. The third class is net neutral in charge (*e.g*., highly crosslinked polyethylene oxide, or polyvinylalcohol).

Any suitable matrix or spacer may be employed within the core, including precipitated chitosan, synthetic polymers and polymer blends, microcarriers and the like, depending upon the growth characteristics of the cells to be encapsulated.

Alternatively, the capsule may have an internal scaffold. The scaffold may prevent cells from aggregating and improve cellular distribution within the device. *(See* PCT publication no. WO 96/02646). The scaffold defines the microenvironment for the encapsulated cells and keeps the cells well distributed within the core. The optimal internal scaffold for a particular device is highly dependent on the cell type to be used. In the absence of such a scaffold, adherent cells aggregate to form clusters.

For example, the internal scaffold may be a yam or a mesh. The filaments used to form a yarn or mesh internal scaffold are formed of any suitable biocompatible, substantially non-degradable material. (*See* United States Patent Nos. 6,303,136 and 6,627,422, which are herein incorporated by reference). Preferably, the capsule of this invention will be similar to those described by PCT International patent applications WO 92/19195 or WO 95/05452, incorporated by reference; or U.S. Pat. Nos. 5,639,275; 5,653,975; 4,892,538; 5,156,844; 5,283,187; or 5,550,050, incorporated by reference. Materials useful in forming yarns or woven meshes include any biocompatible polymers that are able to be formed into fibers such as, for example, acrylic, polyester, polyethylene, polypropylene, polyacrylonitrile, polyethylene terephthalate, nylon, polyamides, polyurethanes, polybutester, or natural fibers such as cotton, silk, chitin or carbon. Any suitable thermoplastic polymer, thermoplastic elastomer, or other synthetic or natural material having fiber-forming properties may be inserted into a pre-fabricated hollow fiber membrane or a hollow cylinder formed from a flat membrane sheet. For example, silk, PET or nylon filaments used for suture materials or in the manufacture of vascular grafts are highly conducive to this type of application. In other embodiments, metal ribbon or wire may be used and woven. Each of these filament materials has well-controlled surface and geometric properties, may be mass produced, and has a long history of implant use. In certain embodiments, the filaments may be "texturized" to provide rough surfaces and "hand-holds" onto which cell projections may attach. The filaments may be coated with extracellular matrix molecules or surface-treated (e.g. plasma irradiation) to enhance cellular adhesion to the filaments.

In some embodiments, the filaments, preferably organized in a non-random unidirectional orientation, are twisted in bundles to form yarns of varying thickness and void volume. Void volume is defined as the spaces existing between filaments. The void volume in the yarn should vary between 20-95%, but is preferably between 50-95%. The preferred void space between the filaments is between 20-200 µm, sufficient to allow the scaffold to be seeded with cells along the length of the yarn and to allow the cells to attach to the filaments. The preferred diameter of the filaments comprising the yarn is between 5-100 µm. These filaments should have sufficient mechanical strength to allow twisting into a bundle to comprise a yarn. The filament cross-sectional shape can vary, with circular, rectangular, elliptical, triangular, and/or star-shaped cross-section being preferred.

Alternatively, the filaments or yarns can be woven into a mesh. The mesh can be produced on a braider using carriers, similar to bobbins, containing monofilaments or multifilaments, which serve to feed either the yarn or filaments into the mesh during weaving. The number of carriers is adjustable and may be wound with the same filaments or a combination of filaments with different compositions and structures. The angle of the braid, defined by the pick count, is controlled by the rotational speed of the carriers and the production speed. In one embodiment, a mandrel is used to produce a hollow tube of mesh. In certain embodiments, the braid is constructed as a single layer, in other embodiments it is a multi-layered structure. The tensile strength of the braid is the linear summation of the tensile strengths of the individual filaments.

In other embodiments, a tubular braid is constructed. The braid can be inserted into a hollow fiber membrane upon which the cells are seeded. Alternatively, the cells can be allowed to infiltrate the wall of the mesh tube to maximize the surface area available for cell attachment. When such cell infiltration occurs, the braid serves both as a cell scaffold matrix and as an inner support for the device. The increase in tensile strength for the braid-supported device is significantly higher than in alternative approaches.

As noted, for implant sites that are not immunologically privileged, such as periocular sites, and other areas outside the anterior chamber (aqueous) and the posterior chamber (vitreous), the capsules are preferably immunoisolatory. Components of the biocompatible material may include a surrounding semipermeable membrane and the internal cell-supporting scaffolding. The transformed cells are preferably seeded onto the scaffolding, which is encapsulated by the permselective membrane, which is described above. Also, bonded fiber structures can be used for cell implantation. (*See* U.S. Pat. No. 5,512,600, incorporated by reference). Biodegradable polymers include those comprised of poly(lactic acid) PLA, poly(lactic-coglycolic acid) PLGA, and poly(glycolic acid) PGA and their equivalents. Foam scaffolds have been used to provide surfaces onto which transplanted cells may adhere (PCT International patent application Ser. No. 98/05304, incorporated by reference). Woven mesh tubes have been used as vascular grafts (PCT International patent application WO 99/52573, incorporated by reference). Additionally, the core can be composed of an immobilizing matrix formed from a hydrogel, which stabilizes the position of the cells. A hydrogel is a 3-dimensional network of cross-linked hydrophilic polymers in the form of a gel, substantially composed of water.

Various polymers and polymer blends can be used to manufacture the surrounding semipermeable membrane, including polyacrylates (including acrylic copolymers), polyvinylidenes, polyvinyl chloride copolymers, polyurethanes, polystyrenes, polyamides, cellulose acetates, cellulose nitrates, polysulfones (including polyether sulfones), polyphosphazenes, polyacrylonitriles, poly(acrylonitrile/covinyl chloride), as well as derivatives, copolymers and mixtures thereof. Preferably, the surrounding semipermeable membrane is a biocompatible semipermeable hollow fiber membrane. Such membranes, and methods of making them are disclosed by U.S. Pat. Nos. 5,284,761 and 5,158,881, incorporated by reference. The surrounding semipermeable membrane is formed from a polyether sulfone hollow fiber, such as those described by U.S. Pat. No. 4,976,859 or U.S. Pat. No. 4,968,733, incorporated by reference. An alternate surrounding semipermeable membrane material is polysulfone.

The capsule can be any configuration appropriate for maintaining biological activity and providing access for delivery of the product or function, including for example, cylindrical, rectangular, disk-shaped, patch-shaped, ovoid, stellate, or spherical. Moreover, the capsule can be coiled or wrapped into a mesh-like or nested structure. If the capsule is to be retrieved after it is implanted, configurations which tend to lead to migration of the capsules from the site of implantation, such as spherical capsules small enough to travel in the recipient host's blood vessels, are not preferred. Certain shapes, such as rectangles, patches, disks, cylinders, and flat sheets offer greater structural integrity and are preferable where retrieval is desired.

Preferably the device has a tether that aids in maintaining device placement during implant, and aids in retrieval. Such a tether may have any suitable shape that is adapted to secure the capsule in place. For example, the suture may be a loop, a disk, or a suture. In some embodiments, the tether is shaped like an eyelet, so that suture may be used to secure the tether (and thus the device) to the sclera, or other suitable ocular structure. In another embodiment, the tether is continuous with the capsule at one end, and forms a pre-threaded suture needle at the other end. In one preferred embodiment, the tether is an anchor loop that is adapted for anchoring the capsule to an ocular structure. The tether may be constructed of a shape memory metal and/or any other suitable medical grade material known in the art.

In a hollow fiber configuration, the fiber will have an inside diameter of less than 1000 microns, preferably less than 750 microns. We also contemplate devices having an outside diameter less than 300-600 microns. For implantation in the eye, in a hollow fiber configuration the capsule will preferably be between 0.4 cm to 1.S cm in length, most preferably between 0.5 to 1.0 cm in length. Longer devices may be accommodated in the eye, however, a curved or accurate shape may be required for secure and appropriate placement. The hollow fiber configuration is preferred for intraocular placement.

For periocular placement, either a hollow fiber configuration (with dimensions substantially as above) or a flat sheet configuration is contemplated. The upper limit contemplated for a flat sheet is approximately 5 mm x 5 mm--assuming a square shape. Other shapes with approximately the same surface area are also contemplated.

The hydraulic permeability will typically be in the range of 1-100 mls/min/M²/mmHg, preferably in the range of 25 to 70 mls/min/M²/mmHg. The glucose mass transfer coefficient of the capsule, defined, measured and calculated as described by Dionne et al., ASAIO Abstracts, p. 99 (1993), and Colton et al., The Kidney, eds., Brenner B M and Rector F C, pp. 2425-89 (1981) will be greater than 10⁻⁶ cm/sec, preferably greater than 10⁻⁴ cm/sec.

The surrounding or peripheral region (jacket), which surrounds the core of the instant devices can be permselective, biocompatible, and/or immunoisolatory. It is produced in such a manner that it is free of isolated cells, and completely surrounds (*i.e*., isolates) the core, thereby preventing contact between any cells in the core and the recipient's body. Biocompatible semi-permeable hollow fiber membranes, and methods of making them are disclosed in U.S. Pat. Nos. 5,284,761 and 5,158,881 (*See also,* WO 95/05452), each of which incorporated herein by reference in its entirety. For example, the capsule jacket can be formed from a polyether sulfone hollow fiber, such as those described in U.S. Pat. Nos. 4,976,859 and 4,968,733, and 5,762,798, each incorporated herein by reference.

To be permselective, the jacket is formed in such a manner that it has a molecular weight cut off ("MWCO") range appropriate both to the type and extent of immunological reaction anticipated to be encountered after the device is implanted and to the molecular size of the largest substance whose passage into and out of the device into the eye is desirable. The type and extent of immunological attacks which may be mounted by the recipient following implantation of the device depend in part upon the type(s) of moiety isolated within it and in part upon the identity of the recipient (*i*.*e*., how closely the recipient is genetically related to the source of the BAM). When the implanted tissue or cells are allogeneic to the recipient, immunological rejection may proceed largely through cell-mediated attack by the recipient's immune cells against the implanted cells. When the tissue or cells are xenogeneic to the recipient, molecular attack through assembly of the recipient's cytolytic complement attack complex may predominate, as well as the antibody interaction with complement.

The jacket allows passage into the eye of substances up to a predetermined size, but prevents the passage of larger substances. More specifically, the surrounding or peripheral region is produced in such a manner that it has pores or voids of a predetermined range of sizes, and, as a result, the device is permselective. The MWCO of the surrounding jacket must be sufficiently low to prevent access of the substances required to carry out immunological attacks to the core, yet sufficiently high to allow delivery of PGF2a to the recipient's eye. Preferably, the MWCO of the biocompatible jacket of the devices of the instant invention is from about 1 kD to about 150 kD.

As used herein with respect to the jacket of the device, the term "biocompatible" refers collectively to both the device and its contents. Specifically, it refers to the capability of the implanted intact device and its contents to avoid the detrimental effects of the body's various protective systems and to remain functional for a significant period of time. As used herein, the term "protective systems" refers to the types of immunological attack which can be mounted by the immune system of an individual in whom the instant vehicle is implanted, and to other rejection mechanisms, such as the fibrotic response, foreign body response and other types of inflammatory response which can be induced by the presence of a foreign object in the individuals' body. In addition to the avoidance of protective responses from the immune system or foreign body fibrotic response, the term "biocompatible", as used herein, also implies that no specific undesirable cytotoxic or systemic effects are caused by the vehicle and its contents such as those that would interfere with the desired functioning of the vehicle or its contents.

The external surface of the device can be selected or designed in such a manner that it is particularly suitable for implantation at a selected site. For example, the external surface can be smooth, stippled, or rough, depending on whether attachment by cells of the surrounding tissue is desirable. The shape or configuration can also be selected or designed to be particularly appropriate for the implantation site chosen.

The biocompatibility of the surrounding or peripheral region (jacket) of the device is produced by a combination of factors. Important for biocompatibility and continued functionality are device morphology, hydrophobicity and the absence of undesirable substances either on the surface of, or leachable from, the device itself. Thus, brush surfaces, folds, interlayers or other shapes or structures eliciting a foreign body response are avoided. Moreover, the device-forming materials are sufficiently pure to insure that unwanted substances do not leach out from the device materials themselves. Additionally, following device preparation, the treatment of the external surface of the device with fluids or materials (*e.g*. serum) which may adhere to or be absorbed by the device and subsequently impair device biocompatibility is avoided.

First, the materials used to form the device jacket are substances selected based upon their ability to be compatible with, and accepted by, the tissues of the recipient of the implanted device. Substances are used which are not harmful to the recipient or to the isolated cells. Preferred substances include polymer materials, *i.e*., thermoplastic polymers. Particularly preferred thermoplastic polymer substances are those which are modestly hydrophobic, *i*.*e*. those having a solubility parameter as defined in Brandrup J., et al. Polymer Handbook 3rd Ed., John Wiley & Sons, NY (1989), between 8 and 15, or more preferably, between 9 and 14 (Joules/m³)^{1/2}. The polymer substances are chosen to have a solubility parameter low enough so that they are soluble in organic solvents and still high enough so that they will partition to form a proper membrane. Such polymer substances should be substantially free of labile nucleophilic moieties and be highly resistant to oxidants and enzymes even in the absence of stabilizing agents. The period of residence *in vivo* which is contemplated for the particular vehicle must also be considered: substances must be chosen which are adequately stable when exposed to physiological conditions and stresses. Many thermoplastics are known which are sufficiently stable, even for extended periods of residence *in vivo,* such as periods in excess of one or two years.

The choice of materials used to construct the device is determined by a number of factors as described in detail in Dionne WO 92/19195, herein incorporated by reference. Briefly, various polymers and polymer blends can be used to manufacture the capsule jacket. Polymeric membranes forming the device and the growth surfaces therein may include polyacrylates (including acrylic copolymers), polyvinylidenes, polyvinyl chloride copolymers, polyurethanes, polystyrenes, polyamides, polymethylmethacrylate, polyvinyldifluoride, polyolefins, cellulose acetates, cellulose nitrates, polysulfones, polyphosphazenes, polyacrylonitriles, poly(acrylonitrile/covinyl chloride), as well as derivatives, copolymers and mixtures thereof.

A preferred membrane casting solution comprises a either a polysulfone dissolved in the water-miscible solvent dimethylacetamide (DMACSO) or polyethersulfone dissolved in the water-miscible solvent butyrolactone. This casting solution can optionally comprise hydrophilic or hydrophobic additives which affect the permeability characteristics of the finished membrane. A preferred hydrophilic additive for the polysulfone or polyethersulfone is polyvinylpyrrolidone (PVP). Other suitable polymers comprise polyacrylonitrile (PAN), polymethylmethacrylate (PMMA), polyvinyldifluoride (PVDF), polyethylene oxide, polyolefins (*e.g*., polyisobutylene or polypropylene), polyacrylonitrile/polyvinyl chloride (PAN/PVC), and/or cellulose derivatives (*e.g*., cellulose acetate or cellulose butyrate). Compatible water-miscible solvents for these and other suitable polymers and copolymers are found in the teachings of U.S. Pat. No. 3,615,024.

Second, substances used in preparing the biocompatible jacket of the device are either free of leachable pyrogenic or otherwise harmful, irritating, or immunogenic substances or are exhaustively purified to remove such harmful substances. Thereafter, and throughout the manufacture and maintenance of the device prior to implantation, great care is taken to prevent the adulteration or contamination of the device or jacket with substances, which would adversely affect its biocompatibility.

Third, the exterior configuration of the device, including its texture, is formed in such a manner that it provides an optimal interface with the eye of the recipient after implantation. Certain device geometries have also been found to specifically elicit foreign body fibrotic responses and should be avoided. Thus, devices should not contain structures having interlayers such as brush surfaces or folds. In general, opposing vehicle surfaces or edges either from the same or adjacent vehicles should be at least 1 mm apart, preferably greater than 2 mm and most preferably greater than 5 mm. Preferred embodiments include cylinders having an outer diameter of between about 200 and 350 µm and a length between about 0.5 and 6 mm. Preferably, the core of the devices of the invention has a volume of approximately 2.5 µl. However, those skilled in the art will recognize that it is also possible to use "micronized" devices having a core volume of less than 0.5 µl (e*.g.*, about 0.3 µl).

The surrounding jacket of the biocompatible devices can optionally include substances which decrease or deter local inflammatory response to the implanted vehicle and/or generate or foster a suitable local environment for the implanted cells or tissues. For example antibodies to one or more mediators of the immune response could be included. Available potentially useful antibodies such as antibodies to the lymphokines tumor necrosis factor (TNF), and to interferons (IFN) can be included in the matrix precursor solution. Similarly, an anti-inflammatory steroid can be included. See Christenson, L., et al., J. Biomed. Mat. Res., 23, pp. 705-718 (1989); Christenson, L., Ph.D. thesis, Brown University, 1989, herein incorporated by reference. Alternatively, a substance which stimulates angiogenesis (ingrowth of capillary beds) can be included.

In some embodiments, the jacket of the present device is immunoisolatory. That is, it protects cells in the core of the device from the immune system of the individual in whom the device is implanted. It does so (1) by preventing harmful substances of the individual's body from entering the core, (2) by minimizing contact between the individual and inflammatory, antigenic, or otherwise harmful materials which may be present in the core and (3) by providing a spatial and physical barrier sufficient to prevent immunological contact between the isolated moiety and detrimental portions of the individual's immune system.

In some embodiments, the external jacket may be either an ultrafiltration membrane or a microporous membrane. Those skilled in the art will recognize that ultrafiltration membranes are those having a pore size range of from about 1 to about 100 nanometers while a microporous membrane has a range of between about 0.05 to about 10 microns.

The thickness of this physical barrier can vary, but it will always be sufficiently thick to prevent direct contact between the cells and/or substances on either side of the barrier. The thickness of this region generally ranges between 5 and 200 microns; thicknesses of 10 to 100 microns are preferred, and thickness of 20 to 50 or 20 to 75 microns are particularly preferred. Types of immunological attack which can be prevented or minimized by the use of the instant device include attack by macrophages, neutrophils, cellular immune responses (e.g. natural killer cells and antibody-dependent T cell-mediated cytoloysis (ADCC)), and humoral response (e.g. antibody-dependent complement mediated cytolysis).

The capsule jacket may be manufactured from various polymers and polymer blends including polyacrylates (including acrylic copolymers), polyvinylidenes, polyvinyl chloride copolymers, polyurethanes, polystyrenes, polyamides, cellulose acetates, cellulose nitrates, polysulfones (including polyether sulfones), polyphosphazenes, polyacrylonitriles, poly(acrylonitrile/covinyl chloride), as well as derivatives, copolymers and mixtures thereof. Capsules manufactured from such materials are described, *e.g*., in U.S. Pat. Nos. 5,284,761 and 5,158,881, incorporated herein by reference. Capsules formed from a polyether sulfone (PES) fiber, such as those described in U.S. Pat. Nos. 4,976,859 and 4,968,733, incorporated herein by reference, may also be used.

Depending on the outer surface morphology, capsules have been categorized as Type 1 (T1), Type 2 (T2), Type 1/2 (T1/2), or Type 4 (T4). Such membranes are described, *e.g*., in Lacy et al., "Maintenance Of Normoglycemia In Diabetic Mice By Subcutaneous Xenografts Of Encapsulated Islets", Science, 254, pp. 1782-84 (1991), Dionne et al., WO 92/19195 and Baetge, WO 95/05452. A smooth outer surface morphology is preferred.

Those skilled in the art will recognize that capsule jackets with permselective, immunoisolatory membranes are preferable for sites that are not immunologically privileged. In contrast, microporous membranes or permselective membranes may be suitable for immunologically privileged sites. For implantation into immunologically privileged sites, capsules made from the PES or PS (polyether sulfone or polysulfone, respectively) membranes are preferred.

Any suitable method of sealing the capsules know in the art may be used, including the employment of polymer adhesives and/or crimping, knotting and heat sealing. In addition, any suitable "dry" sealing method can also be used. In such methods, a substantially non-porous fitting is provided through which the cell-containing solution is introduced. Subsequent to filling, the capsule is sealed. Such methods are described in, e.g., United States Patent Nos. 5,653,688; 5,713,887; 5,738,673; 6,653,687; 5,932,460; and 6,123,700, which are herein incorporated by reference.

According to the methods of this invention, other molecules may be co-delivered in addition to PGF2a. For example, it may be preferable to deliver a trophic factor(s) with an anti-angiogenic factor.

Co-delivery can be accomplished in a number of ways. First, cells may be transfected with separate constructs containing the genes encoding the described molecules. Second, cells may be transfected with a single construct containing two or more genes as well as the necessary control elements. Third, two or more separately engineered cell lines can be either co-encapsulated or more than one device can be implanted at the site of interest.

Multiple gene expression from a single transcript is preferred over expression from multiple transcription units. *See, e.g.,* Macejak, Nature, 353, pp. 90-94 (1991); WO 94/24870; Mountford and Smith, Trends Genet., 11, pp. 179-84 (1995); Dirks et al., Gene, 128, pp. 247-49 (1993); Martinez-Salas et al., J. Virology, 67, pp. 3748-55 (1993) and Mountford et al., Proc. Natl. Acad. Sci. USA, 91, pp. 4303-07 (1994).

For some indications, it may be preferable to deliver BAMs to two different sites in the eye concurrently. For example, it may be desirable to deliver a neurotrophic factor to the vitreous to supply the neural retina (ganglion cells to the RPE) and to deliver an anti-angiogenic factor via the sub-Tenon's space to supply the choroidal vasculature. The device may also be implanted subconjunctivally to target the retina, vitreous, or anterior chamber of the eye.

The methods and devices of this invention are intended for use in a primate, preferably human host, recipient, patient, subject or individual. A number of different implantation sites are contemplated for the devices and methods of this invention. Suitable implantation sites include, but are not limited to, the aqueous and vitreous humors of the eye, the periocular space, the anterior or posterior chambers, and/or the Subtenon's capsule. The devices of the invention can also be implanted subconjunctivally.

The type and extent of immunological response by the recipient to the implanted device will be influenced by the relationship of the recipient to the isolated cells within the core. For example, if core contains syngeneic cells, these will not cause a vigorous immunological reaction, unless the recipient suffers from an autoimmunity with respect to the particular cell or tissue type within the device. Syngeneic cells or tissue are rarely available. In many cases, allogeneic or xenogeneic cells or tissue (*i*.*e*., from donors of the same species as, or from a different species than, the prospective recipient) may be available. The use of immunoisolatory devices allows the implantation of allogeneic or xenogeneic cells or tissue, without a concomitant need to immunosuppress the recipient. Use of immunoisolatory capsules also allows the use of unmatched cells (allographs). Therefore, the instant device makes it possible to treat many more individuals than can be treated by conventional transplantation techniques.

The type and vigor of an immune response to xenografted tissue is expected to differ from the response encountered when syngeneic or allogeneic tissue is implanted into a recipient. This rejection may proceed primarily by cell-mediated, or by complement-mediated attack. The exclusion of IgG from the core of the vehicle is not the touchstone of immunoprotection, because in most cases IgG alone is insufficient to produce cytolysis of the target cells or tissues. Using immunoisolatory devices, it is possible to deliver needed high molecular weight products or to provide metabolic functions pertaining to high molecular weight substances, provided that critical substances necessary to the mediation of immunological attack are excluded from the immunoisolatory capsule. These substances may comprise the complement attack complex component Clq, or they may comprise phagocytic or cytotoxic cells. Use of immunoisolatory capsules provides a protective barrier between these harmful substances and the isolated cells.

While the devices of the present invention are macrocapsules, those skilled in the art will recognize that microcapsules such as, for example those described in Rha, Lim, and Sun may also be used. *(See,* Rha, C. K. et al., U.S. Pat. No. 4,744,933; Methods in Enzymology 137, pp. 575-579 (1988); U.S. Patent No. 4,652,833; U.S. Patent No. 4,409,331). In general, microcapsules differ from macrocapsules by (1) the complete exclusion of cells from the outer layer of the device, and (2) the thickness of the outer layer of the device. Typically, microcapsules have a volume on the order of 1 µl and contain fewer than 10⁴ cells. More specifically, microencapsulation encapsulates approximately 1-10 viable islets or 500 cells, generally, per capsule.

Capsules with a lower MWCO may be used to further prevent interaction of molecules of the patient's immune system with the encapsulated cells.

Any of the devices used in accordance with the methods described herein must provide, in at least one dimension, sufficiently close proximity of any isolated cells in the core to the surrounding eye tissues of the recipient in order to maintain the viability and function of the isolated cells. However, the diffusional limitations of the materials used to form the device do not in all cases solely prescribe its configurational limits. Certain additives can be used which alter or enhance the diffusional properties, or nutrient or oxygen transport properties, of the basic vehicle. For example, the internal medium of the core can be supplemented with oxygen-saturated perfluorocarbons, thus reducing the needs for immediate contact with blood-borne oxygen. This will allow isolated cells or tissues to remain viable while, for instance, a gradient of angiotensin is released from the vehicle into the surrounding tissues, stimulating ingrowth of capillaries. References and methods for use of perfluorocarbons are given by Faithful, N. S. Anaesthesia, 42, pp. 234-242 (1987) and NASA Tech Briefs MSC-21480, U.S. Govt. Printing Office, Washington, D.C. 20402, incorporated herein by reference. Alternatively for clonal cell lines such as PC12 cells, genetically engineered hemoglobin sequences may be introduced into the cell lines to produce superior oxygen storage. *See* NPO-17517 NASA Tech Briefs, 15, p. 54.

The thickness of the device jacket should be sufficient to prevent an immunoresponse by the patient to the presence of the devices. For that purpose, the devices preferably have a minimum thickness of 1 µm or more and are free of the cells.

Additionally, reinforcing structural elements can also be incorporated into the devices. For example, these structural elements can be made in such a fashion that they are impermeable and are appropriately configured to allow tethering or suturing of the device to the eye tissues of the recipient. In certain circumstances, these elements can act to securely seal the jacket (e.g., at the ends of the cylinder), thereby completing isolation of the core materials (e.g., a molded thermoplastic clip). In many embodiments, it is desirable that these structural elements should not occlude a significant area of the permselective jacket.

The device of the present invention is of a sufficient size and durability for complete retrieval after implantation. The preferred devices of the present invention have a core of approximately 1-3 µl.

Along with PGF2a, at least one additional BAM can be delivered from the device to the eye. For example, the at least one additional BAM can be provided from a cellular or a noncellular source (or a combination thereof). When the at least one additional BAM is provided from a noncellular source, the additional BAM(s) may be encapsulated in, dispersed within, or attached to one or more components of the cell system including, but not limited to: (a) sealant; (b) scaffold; (c) jacket membrane; (d) tether anchor; and/or (e) core media. In such embodiment, co-delivery of the BAM from a noncellular source may occur from the same device as the BAM from the cellular source.

Alternatively, two or more encapsulated cell systems can be used. For example, the least one additional biologically active molecule can be a nucleic acid, a nucleic acid fragment, a peptide, a polypeptide, a peptidomimetic, a carbohydrate, a lipid, an organic molecule, an inorganic molecule, a therapeutic agent, or any combinations thereof. Specifically, the therapeutic agents may be an anti-angiogenic drug, a steroidal and non-steroidal anti-inflammatory drug, an anti-mitotic drug, an anti-tumor drug, an anti-parasitic drug, an IOP reducer, a peptide drug, and/or any other biologically active molecule drugs approved for ophthalmologic use.

Suitable excipients include, but are not limited to, any non-degradable or biodegradable polymers, hydrogels, solubility enhancers, hydrophobic molecules, proteins, salts, or other complexing agents approved for formulations.

Non-cellular dosages can be varied by any suitable method known in the art such as varying the concentration of the therapeutic agent, and/or the number of devices per eye, and/or modifying the composition of the encapsulating excipient. Cellular dosage can be varied by changing (1) the number of cells per device, (2) the number of devices per eye, and/or (3) the level of BAM production per cell. Cellular production can be varied by changing, for example, the copy number of the gene for the BAM in the transduced cell, or the efficiency of the promoter driving expression of the BAM. Suitable dosages from non-cellular sources may range from about 1 pg to about 1000 ng per day.

The instant invention also relates to methods for making the macrocapsular devices described herein. Devices may be formed by any suitable method known in the art. (*See*, *e*.*g*., United States Patent Nos. 6,361,771; 5,639,275; 5,653,975; 4,892,538; 5,156,844; 5,283,138; and 5,550,050, each of which is incorporated herein by reference).

Membranes used can also be tailored to control the diffusion of molecules, such as PGF2a, based on their molecular weight. (*See* Lysaght et al., 56 J. Cell Biochem. 196 (1996), Colton, 14 Trends Biotechnol. 158 (1996)). Using encapsulation techniques, cells can be transplanted into a host without immune rejection, either with or without use of immunosuppressive drugs. The capsule can be made from a biocompatible material that, after implantation in a host, does not elicit a detrimental host response sufficient to result in the rejection of the capsule or to render it inoperable, for example through degradation. The biocompatible material is relatively impermeable to large molecules, such as components of the host's immune system, but is permeable to small molecules, such as insulin, growth factors, and nutrients, while allowing metabolic waste to be removed. A variety of biocompatible materials are suitable for delivery of growth factors by the composition of the invention. Numerous biocompatible materials are known, having various outer surface morphologies and other mechanical and structural characteristics.

If a device with a jacket of thermoplastic or polymer membrane is desired, the pore size range and distribution can be determined by varying the solids content of the solution of precursor material (the casting solution), the chemical composition of the water-miscible solvent, or optionally including a hydrophilic or hydrophobic additive to the casting solution, as taught by U.S. Pat. No. 3,615,024. The pore size may also be adjusted by varying the hydrophobicity of the coagulant and/or of the bath.

Typically, the casting solution will comprise a polar organic solvent containing a dissolved, water-insoluble polymer or copolymer. This polymer or copolymer precipitates upon contact with a solvent-miscible aqueous phase, forming a permselective membrane at the site of interface. The size of pores in the membrane depends upon the rate of diffusion of the aqueous phase into the solvent phase; the hydrophilic or hydrophobic additives affect pore size by altering this rate of diffusion. As the aqueous phase diffuses farther into the solvent, the remainder of the polymer or copolymer is precipitated to form a trabecular support which confers mechanical strength to the finished device.

The external surface of the device is similarly determined by the conditions under which the dissolved polymer or copolymer is precipitated (*i*.*e*., exposed to the air, which generates an open, trabecular or sponge-like outer skin, immersed in an aqueous precipitation bath, which results in a smooth permselective membrane bilayer, or exposed to air saturated with water vapor, which results in an intermediate structure).

The surface texture of the device is dependent in part on whether the extrusion nozzle is positioned above, or immersed in, the bath: if the nozzle is placed above the surface of the bath a roughened outer skin of PAN/PVC will be formed, whereas if the nozzle is immersed in the bath a smooth external surface is formed.

The surrounding or peripheral matrix or membrane can be preformed, filled with the materials which will form the core (for instance, using a syringe), and subsequently sealed in such a manner that the core materials are completely enclosed. The device can then be exposed to conditions which bring about the formation of a core matrix if a matrix precursor material is present in the core.

The devices of the invention can provide for the implantation of diverse cell or tissue types, including fully-differentiated, anchorage-dependent, fetal or neonatal, or transformed, anchorage-independent cells or tissue. The cells to be isolated are prepared either from a donor (*i*.*e*., primary cells or tissues, including adult, neonatal, and fetal cells or tissues) or from cells which replicate *in vitro* (*i*.*e*., immortalized cells or cell lines, including genetically modified cells). In all cases, a sufficient quantity of cells to produce effective levels of the needed product or to supply an effective level of the needed metabolic function is prepared, generally under sterile conditions, and maintained appropriately (*e*.*g*. in a balanced salt solution such as Hank's salts, or in a nutrient medium, such as Ham's F12) prior to isolation.

The ECT devices of the invention are of a shape which tends to reduce the distance between the center of the device and the nearest portion of the jacket for purposes of permitting easy access of nutrients from the patient into the cell or of entry of the patient's proteins into the cell to be acted upon by the cell to provide a metabolic function. In that regard, a non-spherical shape, such as a cylinder, is preferred.

Four important factors that influence the number of cells or amount of tissue to be placed within the core of the device (*i*.*e*., loading density) of the instant invention are: (1) device size and geometry; (2) mitotic activity within the device; (3) viscosity requirements for core preparation and or loading; and (4) pre-implantation assay and qualification requirements.

With respect to the first of these factors, (device size and geometry), the diffusion of critical nutrients and metabolic requirements into the cells as well as diffusion of metabolites away from the cell are critical to the continued viability of the cells. In the case of RPE cells such as ARPE-19 cells, the neighboring cells are able to phagocytize the dying cells and use the debris as an energy source.

Among the metabolic requirements met by diffusion of substances into the device is the requirement for oxygen. The oxygen requirements of the specific cells must be determined for the cell of choice. *See* Methods and references for determination of oxygen metabolism are given in Wilson D. F. et al., J. Biol. Chem., 263, pp. 2712-2718, (1988).

With respect to the second factor (cell division), if the cells selected are expected to be actively dividing while in the device, then they will continue to divide until they fill the available space, or until phenomena such as contact inhibition limit further division. For replicating cells, the geometry and size of the device will be chosen so that complete filling of the device core will not lead to deprivation of critical nutrients due to diffusional limitations.

With respect to the third factor (viscosity of core materials) cells in densities occupying up to 70% of the device volume can be viable, but cell solutions in this concentration range would have considerable viscosity. Introduction of cells in a very viscous solution into the device could be prohibitively difficult. In general, for both two step and coextrusion strategies, cell loading densities of higher than 30% will seldom be useful, and in general optimal loading densities will be 20% and below. For example, for fragments of tissues, it is important, in order to preserve the viability of interior cells, to observe the same general guidelines as above and tissue fragments should not exceed 250 microns in diameter with the interior cells having less than 15, preferably less than 10 cells between them and the nearest diffusional surface.

Finally, with respect to the fourth factor (preimplantation and assay requirements), in many cases, a certain amount of time will be required between device preparation and implantation. For instance, it may be important to qualify the device in terms of its biological activity. Thus, in the case of mitotically active cells, preferred loading density will also consider the number of cells which must be present in order to perform the qualification assay.

In most cases, prior to implantation *in vivo,* it will be important to use *in vitro* assays to establish the efficacy of the BAM (*e.g*., PGF2a) within the device. Devices can be constructed and analyzed using model systems in order to allow the determination of the efficacy of the vehicle on a per cell or unit volume basis.

Following these guidelines for device loading and for determination of device efficacy, the actual device size for implantation will then be determined by the amount of biological activity required for the particular application. The number of devices and device size should be sufficient to produce a therapeutic effect upon implantation and is determined by the amount of biological activity required for the particular application. In the case of secretory cells releasing therapeutic substances, standard dosage considerations and criteria known to the art will be used to determine the amount of secretory substance required. Factors to be considered include the size and weight of the recipient; the productivity or functional level of the cells; and, where appropriate, the normal productivity or metabolic activity of the organ or tissue whose function is being replaced or augmented. It is also important to consider that a fraction of the cells may not survive the immunoisolation and implantation procedures. Moreover, whether the recipient has a preexisting condition which can interfere with the efficacy of the implant must also be considered. Devices of the instant invention can easily be manufactured which contain many thousands of cells (*e.g*., between about 5x10² and about 500,000 cells). For example, current clinical devices contain between 200,000 and 400,000 cells, whereas micronized devices would contain between 10,000 and 100,000 cells.

Encapsulated cell therapy is based on the concept of isolating cells from the recipient host's immune system by surrounding the cells with a semipermeable biocompatible material before implantation within the host. For example, the invention includes a device in which genetically engineered ARPE-19 cells are encapsulated in an immunoisolatory capsule, which, upon implantation into a recipient host, minimizes the deleterious effects of the host's immune system on the ARPE-19 cells in the core of the device. ARPE-19 cells are immunoisolated from the host by enclosing them within implantable polymeric capsules formed by a microporous membrane. This approach prevents the cell-to-cell contact between the host and implanted tissues, thereby eliminating antigen recognition through direct presentation.

Delivery of PGF2a using Encapsulated Cell Therapy ("ECT") should overcome the various limitations of topical PGA therapy. The advantage ofECT delivery lies in its ability to deliver a low, continuous, and therapeutic dose of a drug. Moreover, because it is administered directly to the vitreous, ECT can deliver an effective, yet much lower, does than bolus dosing of drops, thereby potentially avoiding untoward side effects. Additionally, ECT provides constant and continuous even dosing, which can potentially eliminate IOP fluctuations.

Using the methods and devices described herein, PGF2a can be delivered intraocularly (*e.g*., in the anterior chamber and the vitreous cavity) or periocularly (*e.g*., within or beneath Tenon's capsule), or both. The devices of the invention may also be used to provide controlled and sustained release of the receptors to treat various ophthalmic disorders, ophthalmic diseases and/or diseases which have ocular effects.

Intraocularly, preferably in the vitreous, delivery of PGF2a in a dosage range of 50 pg to 500 ng, preferably 100 pg to 100 ng, and most preferably 1 ng to 50 ng per eye per patient per day is contemplated. For periocular delivery, preferably in the sub-Tenon's space or region, slightly higher dosage ranges up to 1 µg per patient per day are contemplated. In one preferred embodiment, the delivery of about 1 ng to about 20 ng/device/day of PGF2a (in vivo) is contemplated.

Ophthalmic disorders that may be treated by various embodiments of the present invention include, but are not limited to glaucoma (*e.g*., open angle glaucoma).

Those skilled in the art will recognized that age-related macular degeneration includes, but is not limited to, wet and dry age-related macular degeneration, exudative age-related macular degeneration, and myopic degeneration.

In a preferred embodiment, the disorder to be treated is glaucoma, *e.g*., open angle glaucoma. Those skilled in the art will recognize that glaucoma is a disease characterized by elevated intraocular pressure. Topical administration of PGF2a has been shown to lower IOP but such treatment is often accompanied by unacceptable side effects. Thus, the use of ECT to deliver PGF2a will lower and/or stabilize IOP in patients suffering from glaucoma.

Glaucoma is part of a group of diseases of the optic nerve that involve loss of retinal ganglion cells in a characteristic pattern of optic neuropathy. Raised intraocular pressure (*e.g*. above 22 mmHg) can be a significant risk factor for developing glaucoma. However, one person may develop nerve damage at a relatively low pressure, while another person may have high eye pressure for years and yet never develop damage. Untreated glaucoma leads to permanent damage of the optic nerve and resultant visual field loss, which can progress to blindness.

Glaucoma can be divided into two main categories: "open angle" or chronic glaucoma and "closed angle" or acute glaucoma. Angle closure, *i*.*e*. acute glaucoma, appears suddenly, often with painful side effects, and is usually diagnosed quickly, although damage and loss of vision occurs very suddenly. Open angle, *i*.*e*. chronic glaucoma, tends to progress more slowly, and the patient may not notice symptoms until the disease has progressed quite significantly. With either form, once the visual field is lost, the damage can never be reversed.

The major risk factor for most glaucomas is increased intraocular pressure. Intraocular pressure is a function of production of liquid aqueous humor by the ciliary body of the eye and its drainage through the trabecular meshwork. Aqueous humor flows from the ciliary bodies into the posterior chamber, bounded posteriorly by the lens and the zonule of Zinn and anteriorly by the iris. Aqueous humor then flows through the pupil of the iris into the anterior chamber, bounded posteriorly by the iris and anteriorly by the cornea. From here the trabecular meshwork drains aqueous humor via Schlemm's canal into scleral plexuses and general blood circulation. In open angle glaucoma there is reduced flow through the trabecular meshwork; in angle closure glaucoma, the iris is pushed forward against the trabecular meshwork, blocking fluid from escaping.

The inconsistent relationship of glaucomatous optic neuropathy with ocular hypertension relates to anatomic structure, eye development, nerve compression trauma, optic nerve blood flow, excitatory neurotransmitter, trophic factor, retinal ganglion cell/axon degeneration, glial support cell, immune, and aging mechanisms of neuron loss.

The use of the devices and techniques described herein provide several advantages over other delivery routes: PGF2a can be delivered to the eye directly, which reduces or minimizes unwanted peripheral side effects and very small doses of the BAM (*i*.*e*., nanogram or low microgram quantities rather than milligrams) can be delivered compared with topical applications, thereby also potentially lessening side effects. Moreover, since viable cells continuously produce newly synthesized PGF2a, these techniques should be superior to injection or topical delivery of PGF2a, where the dose fluctuates greatly between administrations and the BAM is continuously degraded but not continuously replenished. Thus, it is contemplated that the use of ECT to deliver PGF2a will overcome some (or all) of the problems associated with other PGF2a therapies. Specifically, because ECT is able to deliver low, continuous, and therapeutic dosages of PGF2a (and the PGF2a can be delivered directly to the vitreous of the eye), the unwanted side effects can be reduced or eliminated. Moreover, because ECT provides constant (and continuous dosing), IOP fluctuations in glaucoma patients can be avoided.

Living cells and cell lines genetically engineered to express hCox-2, which, in turn, upregulates PGF2a production, can be encapsulated in the device of the invention and surgically inserted (under retrobulbar anesthesia) into any appropriate anatomical structure of the eye. For example, the devices can be surgically inserted into the vitreous of the eye, where they are preferably tethered to the sclera to aid in removal. Devices can remain in the vitreous as long as necessary to achieve the desired prophylaxis or therapy. For example, the desired therapy may include promotion of neuron or photoreceptor survival or repair, or inhibition and/or reversal of retinal or choroidal neovascularization, as well as inhibition of uveal, retinal and optic nerve inflammation. With vitreal placement, PGF2a may be delivered to the retina or the retinal pigment epithelium (RPE).

In other embodiments, cell-loaded devices are implanted periocularly, within or beneath the space known as Tenon's capsule, which is less invasive than implantation into the vitreous. Therefore, complications such as vitreal hemorrhage and/or retinal detachment are potentially eliminated. This route of administration also permits delivery of PGF2a to the RPE or the retina. Periocular implantation is especially preferred for treating choroidal neovascularization and inflammation of the optic nerve and uveal tract. In general, delivery from periocular implantation sites will permit circulation of PGF2a to the choroidal vasculature, retinal vasculature, and the optic nerve.

Implantation of the biocompatible devices of the invention is performed under sterile conditions. The device can be implanted using a syringe or any other method known to those skilled in the art. Generally, the device is implanted at a site in the recipient's body which will allow appropriate delivery of the secreted product or function to the recipient and of nutrients to the implanted cells or tissue, and will also allow access to the device for retrieval and/or replacement. A number of different implantation sites are contemplated. These include, e.g., the aqueous humor, the vitreous humor, the sub-Tenon's capsule, the periocular space, and the anterior chamber. Preferably, for implant sites that are not immunologically privileged, such as periocular sites, and other areas outside the anterior chamber (aqueous) and the posterior chamber (vitreous), the capsules are immunoisolatory.

It is preferable to verify that the cells immobilized within the device function properly both before and after implantation. Any assays or diagnostic tests well known in the art can be used for these purposes. For example, an ELISA (enzyme-linked immunosorbent assay), chromatographic or enzymatic assay, or bioassay specific for the secreted product can be used. If desired, secretory function of an implant can be monitored over time by collecting appropriate samples (e.g., serum) from the recipient and assaying them.

The use of many of the prior art devices and surgical techniques resulted in a large number of retinal detachments. The occurrence of this complication is lessened because the devices and methods of this invention are less invasive compared to several other therapies.

Any modified, truncated and/or mutein forms of hCox-2 and/or PGF2a known in the art can also be used in accordance with this invention. By way of non-limiting example, Cox-2 like molecules can include compounds that have molecularly evolved from a Cox-2 template; domain shuffled Cox-2 compounds that still retain Cox-2 biological activities (*e.g*., the upregulation of PGF2a production); and/or Cox-2 orthologs/paralogs from a very distant phylogenetic species (*e.g*., jellyfish). Further, the use of active fragments of these receptors (*i.e*., those fragments having biological activity sufficient to achieve a therapeutic effect) is also contemplated. Also contemplated are receptor molecules modified by attachment of one or more polyethylene glycol (PEG) or other repeating polymeric moieties as well as combinations of these proteins and polycistronic versions thereof.

Treatment of many conditions according to the methods described herein will require only one or at most less than 50 implanted devices per eye to supply an appropriate therapeutic dose. Therapeutic dosages may be between about 0.1 µg and 1000 ng per eye per patient per day (*e.g*., between 0.1 µg and 500 ng per eye per patient per day; between 0.1 µg and 250 ng, between 0.1 µg and 100 ng, between 0.1 µg and 50 ng, between 0.1 µg and 25 ng, between 0.1 µg and 10 ng, or between 0.1 pg and 5 ng per eye per patient per day). For example, between about 1-20 ng/device/day can be delivered to the eye per device per day. Each of the devices of the present invention is capable of storing between about 1,000 and about 500,000 cells, in individual or cluster form, depending on their type.

The invention will be further described in the following examples, which do not limit the scope of the invention described in the claims.

### EXAMPLES

### Example 1: Subcloning

The cDNA for human cyclooxygenase 2, hCox-2, (GenBank Accession No. NM_000963.1) was amplified by PCR using oligonucleotide primer pairs specific for the desired product. Amplified products were digested with the appropriate restriction endonuclease and ligated into Neurotech mammalian expression vector pKAN3, a schematic of which is shown in Figure 1. The pKAN3 backbone is based on the pNUT-IgSP-hCNTF expression plasmid used to create the ARPE-19-hCNTF cell lines.

The nucleotide sequence of pKAN3 is shown below:

1 CTTGGTTTTT AAAACCAGCC TGGAGTAGAG CAGATGGGTT AAGGTGAGTG ACCCCTCAGC
GAACCAAAAA TTTTGGTCGG ACCTCATCTC GTCTACCCAA TTCCACTCAC TGGGGAGTCG
61 CCTGGACATT CTTAGATGAG CCCCCTCAGG AGTAGAGAAT AATGTTGAGA TGAGTTCTGT
GGACCTGTAA GAATCTACTC GGGGGAGTCC TCATCTCTTA TTACAACTCT ACTCAAGACA
121 TGGCTAAAAT AATCAAGGCT AGTCTTTATA AAACTGTCTC CTCTTCTCCT AGCTTCGATC
ACCGATTTTA TTAGTTCCGA TCAGAAATAT TTTGACAGAG GAGAAGAGGA TCGAAGCTAG
181 CAGAGAGAGA CCTGGGCGGA GCTGGTCGCT GCTCAGGAAC TCCAGGAAAG GAGAAGCTGA
GTCTCTCTCT GGACCCGCCT CGACCAGCGA CGAGTCCTTG AGGTCCTTTC CTCTTCGACT
241 GGTTACCACG CTGCGAATGG GTTTACGGAG ATAGCTGGCT TTCCGGGGTG AGTTCTCGTA
CCAATGGTGC GACGCTTACC CAAATGCCTC TATCGACCGA AAGGCCCCAC TCAAGAGCAT
301 AACTCCAGAG CAGCGATAGG CCGTAATATC GGGGAAAGCA CTATAGGGAC ATGATGTTCC
TTGAGGTCTC GTCGCTATCC GGCATTATAG CCCCTTTCGT GATATCCCTG TACTACAAGG
361 ACACGTCACA TGGGTCGTCC TATCCGAGCC AGTCGTGCCA AAGGGGCGGT CCCGCTGTGC
TGTGCAGTGT ACCCAGCAGG ATAGGCTCGG TCAGCACGGT TTCCCCGCCA GGGCGACACG
421 ACACTGGCGC TCCAGGGAGC TCTGCACTCC GCCCGAAAAG TGCGCTCGGC TCTGCCAAGG
TGTGACCGCG AGGTCCCTCG AGACGTGAGG CGGGCTTTTC ACGCGAGCCG AGACGGTTCC
481 ACGCGGGGCG CGTGACTATG CGTGGGCTGG AGCAACCGCC TGCTGGGTGC AAACCCTTTG
TGCGCCCCGC GCACTGATAC GCACCCGACC TCGTTGGCGG ACGACCCACG TTTGGGAAAC
541 CGCCCGGACT CGTCCAACGA CTATAAAGAG GGCAGGCTGT CCTCTAAGCG TCACCCCTAG
GCGGGCCTGA GCAGGTTGCT GATATTTCTC CCGTCCGACA GGAGATTCGC AGTGGGGATC
601 AGTCGAGCTG TGACGGTCCT TACAGTCGAG GGCTCGCATC TCTCCTTCAC GCGCCCGCCG
TCAGCTCGAC ACTGCCAGGA ATGTCAGCTC CCGAGCGTAG AGAGGAAGTG CGCGGGCGGC
661 CCCTACCTGA GGCCGCCATC CACGCCGGTT GAGTCGCGTT CTGCCGCCTC CCGCCTGTGG
GGGATGGACT CCGGCGGTAG GTGCGGCCAA CTCAGCGCAA GACGGCGGAG GGCGGACACC
721 TGCCTCCTGA ACTGCGTCCG CCGTCTAGGT AAGTTTAAAG CTCAGGTCGA GACCGGGCCT
ACGGAGGACT TGACGCAGGC GGCAGATCCA TTCAAATTTC GAGTCCAGCT CTGGCCCGGA

781 TTGTCCGGCG CTCCCTTGGA GCCTACCTAG ACTCAGCCGG CTCTCCACGC TTTGCCTGAC
AACAGGCCGC GAGGGAACCT CGGATGGATC TGAGTCGGCC GAGAGGTGCG AAACGGACTG
841 CCTGCTTGCT CAACTCTACG TCTTTGTTTC GTTTTCTGTT CTGCGCCGTT ACAGATCCAA
GGACGAACGA GTTGAGATGC AGAAACAAAG CAAAAGACAA GACGCGGCAA TGTCTAGGTT
901 GCTGTGACCG GCGCCTACCT CGAGACCGGT GCGGCCGCAT TTAAATACTA GTCCGGGTGG
CGACACTGGC CGCGGATGGA GCTCTGGCCA CGCCGGCGTA AATTTATGAT CAGGCCCACC
961 CATCCCTGTG ACCCCTCCCC AGTGCCTCTC CTGGCCCTGG AAGTTGCCAC TCCAGTGCCC
GTAGGGACAC TGGGGAGGGG TCACGGAGAG GACCGGGACC TTCAACGGTG AGGTCACGGG
1021 ACCAGCCTTG TCCTAATAAA ATTAAGTTGC ATCATTTTGT CTGACTAGGT GTCCTTCTAT
TGGTCGGAAC AGGATTATTT TAATTCAACG TAGTAAAACA GACTGATCCA CAGGAAGATA
1081 AATATTATGG GGTGGAGGGG GGTGGTATGG AGCAAGGGGC AAGTTGGGAA GACAACCTGT
TTATAATACC CCACCTCCCC CCACCATACC TCGTTCCCCG TTCAACCCTT CTGTTGGACA
1141 AGGGCCTGCG GGGTCTATTG GGAACCAAGC TGGAGTGCAG TGGCACAATC TTGGCTCACT
TCCCGGACGC CCCAGATAAC CCTTGGTTCG ACCTCACGTC ACCGTGTTAG AACCGAGTGA
1201 GCAATCTCCG CCTCCTGGGT TCAAGCGATT CTCCTGCCTC AGCCTCCCGA CGGCCGTAAT
CGTTAGAGGC GGAGGACCCA AGTTCGCTAA GAGGACGGAG TCGGAGGGCT GCCGGCATTA
1261 TCGTAATCAT GTCATAGCTG TTTCCTGTGT GAAATTGTTA TCCGCTCACA ATTCCACACA
AGCATTAGTA CAGTATCGAC AAAGGACACA CTTTAACAAT AGGCGAGTGT TAAGGTGTGT
1321 ACATACGAGC CGGAAGCATA AAGTGTAAAG CCTGGGGTGC CTAATGAGTG AGCTAACTCA
TGTATGCTCG GCCTTCGTAT TTCACATTTC GGACCCCACG GATTACTCAC TCGATTGAGT
1381 CATTAATTGC GTTGCGCTCA CTGCCCGCTT TCCAGTCGGG AAACCTGTCG TGCCAGCTGC
GTAATTAACG CAACGCGAGT GACGGGCGAA AGGTCAGCCC TTTGGACAGC ACGGTCGACG
1441 ATTAATGAAT CGGCCAACGC GCGGGGAGAG GCGGTTTGCG TATTGGGCGC TCTTCCGCTT
TAATTACTTA GCCGGTTGCG CGCCCCTCTC CGCCAAACGC ATAACCCGCG AGAAGGCGAA
1501 CCTCGCTCAC TGACTCGCTG CGCTCGGTCG TTCGGCTGCG GCGAGCGGTA TCAGCTCACT
GGAGCGAGTG ACTGAGCGAC GCGAGCCAGC AAGCCGACGC CGCTCGCCAT AGTCGAGTGA
1561 CAAAGGCGGT AATACGGTTA TCCACAGAAT CAGGGGATAA CGCAGGAAAG AACATGTGAG
GTTTCCGCCA TTATGCCAAT AGGTGTCTTA GTCCCCTATT GCGTCCTTTC TTGTACACTC
1621 CAAAAGGCCA GCAAAAGGCC AGGAACCGTA AAAAGGCCGC GTTGCTGGCG TTTTTCCATA
GTTTTCCGGT CGTTTTCCGG TCCTTGGCAT TTTTCCGGCG CAACGACCGC AAAAAGGTAT
1681 GGCTCCGCCC CCCTGACGAG CATCACAAAA ATCGACGCTC AAGTCAGAGG TGGCGAAACC
CCGAGGCGGG GGGACTGCTC GTAGTGTTTT TAGCTGCGAG TTCAGTCTCC ACCGCTTTGG
1741 CGACAGGACT ATAAAGATAC CAGGCGTTTC CCCCTGGAAG CTCCCTCGTG CGCTCTCCTG
GCTGTCCTGA TATTTCTATG GTCCGCAAAG GGGGACCTTC GAGGGAGCAC GCGAGAGGAC
1801 TTCCGACCCT GCCGCTTACC GGATACCTGT CCGCCTTTCT CCCTTCGGGA AGCGTGGCGC
AAGGCTGGGA CGGCGAATGG CCTATGGACA GGCGGAAAGA GGGAAGCCCT TCGCACCGCG
1861 TTTCTCATAG CTCACGCTGT AGGTATCTCA GTTCGGTGTA GGTCGTTCGC TCCAAGCTGG
AAAGAGTATC GAGTGCGACA TCCATAGAGT CAAGCCACAT CCAGCAAGCG AGGTTCGACC
1921 GCTGTGTGCA CGAACCCCCC GTTCAGCCCG ACCGCTGCGC CTTATCCGGT AACTATCGTC
CGACACACGT GCTTGGGGGG CAAGTCGGGC TGGCGACGCG GAATAGGCCA TTGATAGCAG
1981 TTGAGTCCAA CCCGGTAAGA CACGACTTAT CGCCACTGGC AGCAGCCACT GGTAACAGGA
AACTCAGGTT GGGCCATTCT GTGCTGAATA GCGGTGACCG TCGTCGGTGA CCATTGTCCT
2041 TTAGCCAGAG CGAGGTATGT AGGCGGTGCT ACAGAGTTCT TGAAGTGGTG GCCTAACTAC
AATCGGTCTC GCTCCATACA TCCGCCACGA TGTCTCAAGA ACTTCACCAC CGGATTGATG
2101 GGCTACACTA GAAGAACAGT ATTTGGTATC TGCGCTCTGC TGAAGCCAGT TACCTTCGGA
CCGATGTGAT CTTCTTGTCA TAAACCATAG ACGCGAGACG ACTTCGGTCA ATGGAAGCCT
2161 AAAAGAGTTG GTAGCTCTTG ATCCGGCAAA CAAACCACCG CTGGTAGCGG TGGTTTTTTT
TTTTCTCAAC CATCGAGAAC TAGGCCGTTT GTTTGGTGGC GACCATCGCC ACCAAAAAAA
2221 GTTTGCAAGC AGCAGATTAC GCGCAGAAAA AAAGGATCTC AAGAAGATCC TTTGATCTTT
CAAACGTTCG TCGTCTAATG CGCGTCTTTT TTTCCTAGAG TTCTTCTAGG AAACTAGAAA
2281 TCTACGGGGT CTGACGCTCA GTGGAACGAA AACTCACGTT AAGGGATTTT GGTCATGAGA
AGATGCCCCA GACTGCGAGT CACCTTGCTT TTGAGTGCAA TTCCCTAAAA CCAGTACTCT
2341 TTATCAAAAA GGATCTTCAC CTAAATCCTT TTAAATTAAA AATGAAGTTT TAAATCAATC
AATAGTTTTT CCTAGAAGTG GATTTAGGAA AATTTAATTT TTACTTCAAA ATTTAGTTAG
2401 TAAAGTATAT ATGAGTAAAC TTGGTCTGAC AGTCTAAGAA ACCATTATTA TCATGACATT
ATTTCATATA TACTCATTTG AACCAGACTG TCAGATTCTT TGGTAATAAT AGTACTGTAA
2461 AACCTATAAA AATAGGCGTA TCACGAGGCC CTTTCGTCTC GCGCGTTTCG GTGATGACGG
TTGGATATTT TTATCCGCAT AGTGCTCCGG GAAAGCAGAG CGCGCAAAGC CACTACTGCC
2521 TGAAAACCTC TGACACATGC AGCTCCCGGA GACGGTCACA GCTTGTCTGT AAGCGGATGC
ACTTTTGGAG ACTGTGTACG TCGAGGGCCT CTGCCAGTGT CGAACAGACA TTCGCCTACG
2581 CGGGAGCAGA CAAGCCCGTC AGGGCGCGTC AGCGGGTGTT GGCGGGTGTC GGGGCTGGCT
GCCCTCGTCT GTTCGGGCAG TCCCGCGCAG TCGCCCACAA CCGCCCACAG CCCCGACCGA
2641 TAACTATGCG GCATCAGAGC AGATTGTACT GAGAGTGCAC CGATCCCCCC GGTACCCGAT
ATTGATACGC CGTAGTCTCG TCTAACATGA CTCTCACGTG GCTAGGGGGG CCATGGGCTA
2701 CCAGACATGA TAAGATACAT TGATGAGTTT GGACAAACCA CAACTAGAAT GCAGTGAAAA
GGTCTGTACT ATTCTATGTA ACTACTCAAA CCTGTTTGGT GTTGATCTTA CGTCACTTTT
2761 AAATGCTTTA TTTGTGAAAT TTGTGATGCT ATTGCTTTAT TTGTAACCAT TATAAGCTGC
TTTACGAAAT AAACACTTTA AACACTACGA TAACGAAATA AACATTGGTA ATATTCGACG
2821 AATAAACAAG TTAACAACAA CAATTGCATT CATTTTATGT TTCAGGTTCA GGGGGAGGTG
TTATTTGTTC AATTGTTGTT GTTAACGTAA GTAAAATACA AAGTCCAAGT CCCCCTCCAC
2881 TGGGAGGTTT TTTAAAGCAA GTAAAACCTC TACAAATGTG GTATGGCTGA TTATGATCAT
ACCCTCCAAA AAATTTCGTT CATTTTGGAG ATGTTTACAC CATACCGACT AATACTAGTA
2941 GAACAGACTG TGAGGACTGA GGGGCCTGAA ATGAGCCTTG GGACTGTGAA TCTAAAATAC
CTTGTCTGAC ACTCCTGACT CCCCGGACTT TACTCGGAAC CCTGACACTT AGATTTTATG
3001 ACAAACAATT AGAATCAGTA GTTTAACACA TTATACACTT AAAAATTTTA TATTTACCTT
TGTTTGTTAA TCTTAGTCAT CAAATTGTGT AATATGTGAA TTTTTAAAAT ATAAATGGAA
3061 AGAGCTTTAA ATCTCTGTAG GTAGTTTGTC CAATTATGTC ACACCACAGA AGTAAGGTTC

TCTCGAAATT TAGAGACATC CATCAAACAG GTTAATACAG TGTGGTGTCT TCATTCCAAG
3121 CTTCACAAAG ATCCCAAGTC GAACCCCAGA GTCCCGCTCA GAAGAACTCG TCAAGAAGGC
GAAGTGTTTC TAGGGTTCAG CTTGGGGTCT CAGGGCGAGT CTTCTTGAGC AGTTCTTCCG
3181 GATAGAAGGC GATGCGCTGC GAATCGGGAG CGGCGATACC GTAAAGCACG AGGAAGCGGT
CTATCTTCCG CTACGCGACG CTTAGCCCTC GCCGCTATGG CATTTCGTGC TCCTTCGCCA
3241 CAGCCCATTC GCCGCCAAGC TCTTCAGCAA TATCACGGGT AGCCAACGCT ATGTCCTGAT
GTCGGGTAAG CGGCGGTTCG AGAAGTCGTT ATAGTGCCCA TCGGTTGCGA TACAGGACTA
3301 AGCGGTCCGC CACACCCAGC CGGCCACAGT CGATGAATCC AGAAAAGCGG CCATTTTCCA
TCGCCAGGCG GTGTGGGTCG GCCGGTGTCA GCTACTTAGG TCTTTTCGCC GGTAAAAGGT
3361 CCATGATATT CGGCAAGCAG GCATCGCCAT GGGTCACGAC GAGATCCTCG CCGTCGGGCA
GGTACTATAA GCCGTTCGTC CGTAGCGGTA CCCAGTGCTG CTCTAGGAGC GGCAGCCCGT
3421 TGCGCGCCTT GAGCCTGGCG AACAGTTCGG CTGGCGCGAG CCCCTGATGC TCTTCGTCCA
ACGCGCGGAA CTCGGACCGC TTGTCAAGCC GACCGCGCTC GGGGACTACG AGAAGCAGGT
3481 GATCATCCTG ATCGACAAGA CCGGCTTCCA TCCGAGTACG TGCTCGCTCG ATGCGATGTT
CTAGTAGGAC TAGCTGTTCT GGCCGAAGGT AGGCTCATGC ACGAGCGAGC TACGCTACAA
3541 TCGCTTGGTG GTCGAATGGG CAGGTAGCCG GATCAAGCGT ATGCAGCCGC CGCATTGCAT
AGCGAACCAC CAGCTTACCC GTCCATCGGC CTAGTTCGCA TACGTCGGCG GCGTAACGTA
3601 CAGCCATGAT GGATACTTTC TCGGCAGGAG CAAGGTGAGA TGACAGGAGA TCCTGCCCCG
GTCGGTACTA CCTATGAAAG AGCCGTCCTC GTTCCACTCT ACTGTCCTCT AGGACGGGGC
3661 GCACTTCGCC CAATAGCAGC CAGTCCCTTC CCGCTTCAGT GACAACGTCG AGCACAGCTG
CGTGAAGCGG GTTATCGTCG GTCAGGGAAG GGCGAAGTCA CTGTTGCAGC TCGTGTCGAC
3721 CGCAAGGAAC GCCCGTCGTG GCCAGCCACG ATAGCCGCGC TGCCTCGTCC TGCAGTTCAT
GCGTTCCTTG CGGGCAGCAC CGGTCGGTGC TATCGGCGCG ACGGAGCAGG ACGTCAAGTA
3781 TCAGGGCACC GGACAGGTCG GTCTTGACAA AAAGAACCGG GCGCCCCTGC GCTGACAGCC
AGTCCCGTGG CCTGTCCAGC CAGAACTGTT TTTCTTGGCC CGCGGGGACG CGACTGTCGG
3841 GGAACACGGC GGCATCAGAG CAGCCGATTG TCTGTTGTGC CCAGTCATAG CCGAATAGCC
CCTTGTGCCG CCGTAGTCTC GTCGGCTAAC AGACAACACG GGTCAGTATC GGCTTATCGG
3901 TCTCCACCCA AGCGGCCGGA GAACCTGCGT GCAATCCATC TTGTTCAATC ATGCGAAACG
AGAGGTGGGT TCGCCGGCCT CTTGGACGCA CGTTAGGTAG AACAAGTTAG TACGCTTTGC
3961 ATCCTCATCC TGTCTCTTGA TCAGATCCCA AGCTGGGGAT CTGCAGGAAT CGATATCAAG
TAGGAGTAGG ACAGAGAACT AGTCTAGGGT TCGACCCCTA GACGTCCTTA GCTATAGTTC
4021 CTTATCGATA AGCTTTTTGC AAAAGCCTAG GCCTCCAAAA AAGCCTCCTC ACTACTTCTG
GAATAGCTAT TCGAAAAACG TTTTCGGATC CGGAGGTTTT TTCGGAGGAG TGATGAAGAC
4081 GAATAGCTCA GAGGCCGAGG CGGCCTCGGC CTCTGCATAA ATAAAAAAAA TTAGTCAGCC
CTTATCGAGT CTCCGGCTCC GCCGGAGCCG GAGACGTATT TATTTTTTTT AATCAGTCGG
4141 ATGGGGCGGA GAATGGGCGG AACTGGGCGG AGTTAGGGGC GGGATGGGCG GAGTTAGGGG
TACCCCGCCT CTTACCCGCC TTGACCCGCC TCAATCCCCG CCCTACCCGC CTCAATCCCC
4201 CGGGACTATG GTTGCTGACT AATTGAGATG CATGCTTTGC ATACTTCTGC CTGCTGGGGA
GCCCTGATAC CAACGACTGA TTAACTCTAC GTACGAAACG TATGAAGACG GACGACCCCT
4261 GCCTGGGGAC TTTCCACACC TGGTTGCTGA CTAATTGAGA TGCATGCTTT GCATACTTCT
CGGACCCCTG AAAGGTGTGG ACCAACGACT GATTAACTCT ACGTACGAAA CGTATGAAGA
4321 GCCTGCTGGG GAGCCTGGGG ACTTTCCACA CCCTAACTGA CACACATTCC ACAGCTGGTT
CGGACGACCC CTCGGACCCC TGAAAGGTGT GGGATTGACT GTGTGTAAGG TGTCGACCAA
4381 CTTTCCGCCT CAGAAGGTAC ACTCTTCCTT TTTCAATATT ATTGAAGCAT TTATCAGGGT
GAAAGGCGGA GTCTTCCATG TGAGAAGGAA AAAGTTATAA TAACTTCGTA AATAGTCCCA
4441 TATTGTCTCA TGAGCGGATA CATATTTGAA TGTATTTAGA AAAATAAACA AATAGGGGTT
ATAACAGAGT ACTCGCCTAT GTATAAACTT ACATAAATCT TTTTATTTGT TTATCCCCAA
4501 CCGCGCACAT TTCCCCGAAA AGTGCCACCT GACGGCGGCC
GGCGCGTGTA AAGGGGCTTT TCACGGTGGA CTGCCGCCGG (SEQ ID NO:3)

In SEQ ID NO:3, nucleotides 1-595 are pMT1; nucleotides 631-918 are U5 5' UTR, nucleotides 919-953 are MCS; nucleotides 954-1250 are hGH pA; nucleotides 1258-2680 are pUC 18; nucleotides 2698-2992 are SV40 pA; nucleotides 2988-3133 are SV40 Intron E19S; nucleotides 3158-3952 are NeoR; nucleotides 4030-4400 are SV40 promoter; and 4401-4534 are AmpR promoter.

Transformed recombinant clones were selected with kanamycin, and purified miniprep plasmid DNA was analyzed by restriction digestion and agarose gel electrophoresis analysis. Putative plasmid clones containing an appropriate insert were verified by automated dideoxy sequencing followed by alignment analysis using Vector NTI v7.0 sequence analysis software (Invitrogen Corp, Carlsbad, CA).

### Example 2: Cell Line Construction

Verified plasmid clones were used to transfect ARPE-10 cells (*i*.*e*., NTC-200 cells) to obtain stable polyclonal cell lines. Briefly, 200-300K cells, plated 18 hours previously, were transfected with 3.0 ug of plasmid DNA using 6.0 ul of Fugene 6 transfection reagent (Roche Applied Science, Indianapolis IN) according to the manufacturer's recommendations. Transfections were performed in 2.0-3.0 ml of DMEM/F12 with 10% FBS, Endothelial SFM or Optimem media (Invitrogen Corp, Carlsbad, CA). Twenty four to 48 hours later cells were either fed with fresh media containing 1.0 ug/ul of G418 or passaged to a T-25 tissue culture flask containing G418. Cell lines were passaged under selection for 14-21 days until normal growth resumed, after which time drug was removed and cells were allowed to recover (~1 week) prior to characterization.

Stability of production/synthesis of prostaglandin F 2α from these cell lines was measured over the course of several weeks using the PGF2alpha High Sensitivity ELISA Kit (Assay Designs, Ann Arbor, MI). Briefly, 50K cells, previously plated into 12 well tissue culture plates in DMEM/F12 with 10% FBS, were washed twice in HBSS (Invitrogen Corp, Carlsbad, CA) then pulsed for 24 hours with 1.0 ml of DMEM/F12 base media lacking FBS (Invitrogen Corp, Carlsbad, CA). Pulse media was stored at -20 C and assayed within one week of collection as per the manufacturer's protocol.

### EQUIVALENTS

The details of one or more embodiments of the invention are set forth in the accompanying description above. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs.

## Claims

1. A cell line for use in treating ophthalmic disorders, wherein said cell line comprises an ARPE-19 cell genetically engineered to express the human Cox-2 (hCox-2) enzyme encoded by the nucleic acid sequence of SEQ ID NO: 1; and wherein said cell-line produces PGF2a in therapeutically effective quantities and is for implantation into the eye of a patient.

2. The cell line for use of claim 1, wherein said expression of the hCox-2 enzyme upregulates the production of prostaglandin F2 alpha (PGF2a).

3. The cell line for use of claim 2, wherein said cell line expresses from about 1 to about 20 ng/million cells/day of PGF2a.

4. An implantable cell culture device, the device comprising:
a) a core comprising one or more ARPE-19 cells genetically engineered to express the human Cox-2 (hCox-2) enzyme for use in treating ophthalmic disorders, wherein the hCox-2 enzyme is encoded by the nucleic acid of SEQ ID NO:1 and wherein the expression of hCox-2 produces prostaglandin F2 alpha (PGF2a) in the one or more ARPE-19 cells; and
b) a semipermeable membrane surrounding the core, wherein the membrane permits diffusion of PGF2a therethrough.

5. The device of claim 4, wherein said device produces from about 1 to about 20 ng per day of PGF2a.

6. The device of claim 4, wherein the core further comprises a matrix disposed within the semipermeable membrane, preferably wherein the matrix comprises a hydrogel or extracellular matrix components.

7. The device of claim 6, wherein the hydrogel comprises alginate cross-linked with a multivalent ion.

8. The device of claim 6, wherein the matrix comprises a plurality of monofilaments, wherein said monofilaments are a) twisted into a yarn or woven into a mesh or b) twisted into a yarn that is in non-woven strands, and wherein the cells are distributed thereon.

9. The device of claim 8, wherein the monofilaments comprise a biocompatible material selected from the group consisting of acrylic, polyester, polyethylene, polypropylene polyacetonitrile, polyethylene terephthalate, nylon, polyamides, polyurethanes, polybutester, silk, cotton, chitin, carbon, and biocompatible metals.

10. The device of claim 4, wherein the device further comprises a tether anchor, preferably wherein the tether anchor comprises an anchor loop, more preferably wherein the anchor loop is adapted for anchoring the device to an ocular structure.

11. The device of claim 10, wherein the device is for implantation into the eye, preferably wherein the device is for implantation in the vitreous, the aqueous humor, the Subtenon's space, the periocular space, the posterior chamber, or the anterior chamber of the eye, more preferably wherein the device is for implantation in the vitreous of the eye.

12. The device of claim 4, wherein the jacket comprises a permselective, immunoisolatory membrane or a microporous membrane.

13. The device of claim 4, wherein the device is configured as a hollow fiber or a flat sheet.

14. The device of claim 4, wherein at least one additional biologically active molecule is co-delivered from the device.

15. The device of claim 14, wherein the at least one additional biologically active molecule is from a non-cellular source.

16. The device of claim 14, wherein the at least one additional biologically active molecule is from a cellular source, preferably wherein the at least on additional biologically active molecule is produced by one or more genetically engineered ARPE-19 cells in the core.

17. The cell line for use according to claim 1, wherein said therapeutically effective quantity of PGF2a production is from about 1 to about 20 ng per million cells per day.

18. The cell line for use according to claim 17, wherein the ophthalmic disorder is glaucoma, preferably wherein the ophthalmic disorder is open angle glaucoma.

19. The cell line for use according to claim 18, wherein production of PGF2a decreases intraocular pressure, stabilizes intraocular pressure, or both decreases and stabilizes intraocular pressure in the patient.

20. The device of claim 4, wherein the device is for implantation into a target region of a recipient host, wherein the encapsulated one or more ARPE-19 cells secrete PGF2a at the target region.

21. The cell line claim 19, wherein the target region is selected from the group consisting of brain, ventricle, spinal cord, the aqueous and vitreous humors of the eye, and the posterior and anterior chamber of the eye, preferably wherein the target region is selected from the group consisting of the aqueous and vitreous humors of the eye, and the posterior and anterior chamber of the eye.

22. A method for making the implantable cell culture device of claim 4, comprising
a) genetically engineering at least one ARPE-19 cell to express the nucleic acid sequence of SEQ ID NO:1; and
b) encapsulating said genetically modified ARPE-19 cells within a semipermeable membrane.

## Patentansprüche

1. Zelllinie zur Verwendung bei der Behandlung von ophthalmischen Störungen, wobei die Zelllinie eine ARPE-19-Zelle umfasst, die derart gentechnisch verändert worden ist, dass sie das menschliche Cox-2 (hCox-2)-Enzym exprimiert, das durch die Nukleinsäuresequenz von SEQ ID NO: 1 kodiert wird, und wobei die Zelllinie PGF2a in therapeutisch wirksamen Mengen erzeugt und zur Implantierung in das Auge eines Patienten dient.

2. Zelllinie zur Verwendung nach Anspruch 1, bei der die Expression des hCox-2-Enzyms die Erzeugung von Prostaglandin F2 alpha (PGF2a) heraufreguliert.

3. Zelllinie zur Verwendung nach Anspruch 2, wobei die Zelllinie von etwa 1 bis etwa 20 ng/Million Zellen/Tag von PGF2a exprimiert.

4. Implantierbare Zellkulturvorrichtung, wobei die Vorrichtung umfasst:
a) einen Kern, der eine oder mehrere ARPE-19-Zellen, die derart gentechnisch verändert worden sind, dass sie das menschliche Cox-2 (hCox-2)-Enzym exprimieren, zur Verwendung zur Behandlung von ophthalmischen Störungen umfasst, wobei das hCox-2-Enzym durch die Nukleinsäure von SEQ ID NO: 1 kodiert wird, und wobei die Expression von hCox-2 Prostaglandin F2 alpha (PGF2a) in der einen oder den mehreren ARPE-19-Zellen erzeugt, und
b) eine semipermeable Membran, die den Kern umgibt, wobei die Membran die Diffusion von PGF2a durch diese zulässt.

5. Vorrichtung nach Anspruch 4, wobei die Vorrichtung von etwa 1 bis etwa 20 ng pro Tag von PGF2a erzeugt.

6. Vorrichtung nach Anspruch 4, wobei der Kern ferner eine Matrix umfasst, die innerhalb der semipermeablen Membran angeordnet ist, wobei die Matrix vorzugsweise ein Hydrogel oder extrazelluläre Matrixkomponenten umfasst.

7. Vorrichtung nach Anspruch 6, wobei das Hydrogel Alginat umfasst, das mit einem mehrwertigen Ion vernetzt ist.

8. Vorrichtung nach Anspruch 6, wobei die Matrix eine Mehrzahl von Monofilamenten umfasst, wobei die Monofilamente a) zu einem Garn verzwirnt oder zu einem Gewebe verwoben sind oder b) zu einem Garn verzwirnt sind, das in nicht-gewebten Fäden vorliegt, und wobei die Zellen darauf verteilt sind.

9. Vorrichtung nach Anspruch 8, wobei die Monofilamente ein biologisch verträgliches Material umfassen, das aus der Gruppe, bestehend aus Acrylmaterialien, Polyester, Polyethylen, Polypropylen, Polyacetonitril, Polyethylenterephthalat, Nylon, Polyamiden, Polyurethanen, Polybutester, Seide, Baumwolle, Chitin, Kohlenstoff und biologisch verträglichen Metallen, ausgewählt ist.

10. Vorrichtung nach Anspruch 4, wobei die Vorrichtung ferner einen Halteanker umfasst, wobei der Halteanker vorzugsweise eine Ankerschleife umfasst, wobei die Ankerschleife mehr bevorzugt zum Verankern der Vorrichtung an einer Augenstruktur angepasst ist.

11. Vorrichtung nach Anspruch 10, wobei die Vorrichtung zur Implantierung in das Auge dient, wobei die Vorrichtung vorzugsweise zur Implantierung in den Glaskörper, das Kammerwasser, den Subtenon-Raum, den periokulären Raum, die hintere Augenkammer oder die vordere Augenkammer dient, wobei die Vorrichtung mehr bevorzugt zur Implantierung in den Glaskörper des Auges dient.

12. Vorrichtung nach Anspruch 4, wobei der Mantel eine permselektive, immunisolatorische Membran oder eine mikroporöse Membran umfasst.

13. Vorrichtung nach Anspruch 4, wobei die Vorrichtung als Hohlfaser oder als flache Lage ausgebildet ist.

14. Vorrichtung nach Anspruch 4, wobei von der Vorrichtung gleichzeitig mindestens ein zusätzliches biologisch aktives Molekül abgegeben wird.

15. Vorrichtung nach Anspruch 14, wobei das mindestens eine zusätzliche biologisch aktive Molekül von einer nicht-zellulären Quelle stammt.

16. Vorrichtung nach Anspruch 14, wobei das mindestens eine zusätzliche biologisch aktive Molekül von einer zellulären Quelle stammt, wobei das mindestens eine zusätzliche biologisch aktive Molekül vorzugsweise durch eine oder mehrere gentechnisch veränderte ARPE-19-Zellen in dem Kern erzeugt wird.

17. Zelllinie zur Verwendung nach Anspruch 1, wobei die therapeutisch wirksame Menge der PGF2a-Erzeugung von etwa 1 bis etwa 20 ng pro Million Zellen pro Tag beträgt.

18. Zelllinie zur Verwendung nach Anspruch 17, wobei die ophthalmische Störung ein Glaukom ist, wobei die ophthalmische Störung vorzugsweise ein Offenwinkelglaukom ist.

19. Zelllinie zur Verwendung nach Anspruch 18, wobei die Erzeugung von PGF2a in dem Patienten den Augeninnendruck vermindert, den Augeninnendruck stabilisiert oder den Augeninnendruck sowohl vermindert als auch stabilisiert.

20. Vorrichtung nach Anspruch 4, wobei die Vorrichtung zur Implantierung in einen Zielbereich eines Empfängers dient, wobei die eine oder die mehreren ARPE-19-Zellen, die eingekapselt sind, PGF2a an dem Zielbereich sekretieren.

21. Zelllinie nach Anspruch 19, wobei der Zielbereich aus der Gruppe, bestehend aus Gehirn, Ventrikel, Rückenmark, Kammerwasser und Glaskörper des Auges, und der hinteren und vorderen Kammer des Auges, ausgewählt ist, wobei der Zielbereich vorzugsweise aus der Gruppe, bestehend aus Kammerwasser und Glaskörper des Auges, und der hinteren und vorderen Kammer des Auges, ausgewählt ist.

22. Verfahren zur Herstellung der implantierbaren Zellkulturvorrichtung nach Anspruch 4, welches
a) das genetische Verändern mindestens einer ARPE-19-Zelle, so dass sie die Nukleinsäuresequenz von SEQ ID NO: 1 exprimiert, und
b) das Einkapseln der genetisch modifizierten ARPE-19-Zellen innerhalb einer semipermeablen Membran
umfasst.

## Revendications

1. Lignée cellulaire pour utilisation dans le traitement de troubles ophtalmiques, laquelle lignée cellulaire comprend une cellule PE-19 génétiquement modifiée pour exprimer l'enzyme Cox-2 humaine (hCox-2) codée par la séquence d'acide nucléique de la SEQ ID NO : 1 ; et laquelle lignée cellulaire produit PGF2a en des quantités efficaces du point de vue thérapeutique et est destinée à être implantée dans l'oeil d'un patient.

2. Lignée cellulaire pour utilisation selon la revendication 1, dans laquelle ladite expression de l'enzyme hCox-2 régule à la hausse la production de prostaglandine F2 alpha (PGF2a).

3. Lignée cellulaire pour utilisation selon la revendication 2, laquelle lignée cellulaire exprime d'environ 1 à environ 20 ng/million de cellules/jour de PGF2a.

4. Dispositif de culture cellulaire implantable, lequel dispositif comprend :
a) un coeur comprenant une ou plusieurs cellule(s) ARPE-19 génétiquement modifiée(s) pour exprimer l'enzyme Cox-2 humaine (hCox-2) pour utilisation dans le traitement de troubles ophtalmiques, l'enzyme hCox-2 étant codée par l'acide nucléique de la SEQ ID NO : 1, et l'expression de hCox-2 produisant de la prostaglandine F2 alpha (PGF2a) dans la ou les cellules ARPE-19 ; et
b) une membrane semi-perméable entourant le coeur, la membrane permettant la diffusion de PGF2a à travers elle.

5. Dispositif selon la revendication 4, lequel dispositif produit d'environ 1 à environ 20 ng par jour de PGF2a.

6. Dispositif selon la revendication 4, dans lequel le coeur comprend en outre une matrice disposée à l'intérieur de la membrane semi-perméable, de préférence dans lequel la matrice comprend un hydrogel ou des composants de matrice extracellulaire.

7. Dispositif selon la revendication 6, dans lequel l'hydrogel comprend un alginate réticulé avec un ion multivalent.

8. Dispositif selon la revendication 6, dans lequel la matrice comprend une pluralité de monofilaments, lesquels monofilaments étant a) torsadés en un fil ou tissés en un filet ou b) torsadés en un fil qui est en brins non tissés, et dans lequel les cellules sont distribuées sur celle-ci.

9. Dispositif selon la revendication 8, dans lequel les monofilaments comprennent un matériau biocompatible choisi dans l'ensemble constitué par l'acrylique, le polyester, le polyéthylène, le polypropylène, le polyacétonitrile, le poly(téréphtalate d'éthylène), le nylon, les polyamides, les polyuréthanes, le polybutester, la soie, le coton, la chitine, le carbone, et les métaux biocompatibles.

10. Dispositif selon la revendication 4, lequel dispositif comprend en outre une ancre d'amarrage, de préférence dans lequel l'ancre d'amarrage comprend une boucle d'ancrage, plus préférablement dans lequel la boucle d'ancrage est adaptée pour ancrer le dispositif à une structure oculaire.

11. Dispositif selon la revendication 10, lequel dispositif est destiné à être implanté dans l'oeil, de préférence lequel dispositif est destiné à être implanté dans le vitré, l'humeur aqueuse, l'espace sous-ténonien, l'espace périoculaire, la chambre postérieure, ou la chambre antérieure de l'oeil, plus préférablement lequel dispositif est destiné à être implanté dans le vitré de l'oeil.

12. Dispositif selon la revendication 4, dans lequel la chemise comprend une membrane permsélective immuno-isolante ou une membrane microporeuse.

13. Dispositif selon la revendication 4, lequel dispositif est configuré sous la forme d'une fibre creuse ou d'une feuille plate.

14. Dispositif selon la revendication 4, dans lequel au moins une molécule biologiquement active additionnelle est co-délivrée à partir du dispositif.

15. Dispositif selon la revendication 14, dans lequel l'au moins une molécule biologiquement active additionnelle est issue d'une source non cellulaire.

16. Dispositif selon la revendication 14, dans lequel l'au moins une molécule biologiquement active additionnelle est issue d'une source cellulaire, de préférence dans lequel l'au moins une molécule biologiquement active additionnelle est produite par une ou plusieurs cellules PE-19 génétiquement modifiées dans le coeur.

17. Lignée cellulaire pour utilisation selon la revendication 1, dans laquelle ladite quantité efficace du point de vue thérapeutique de production de PGF2a est d'environ 1 à environ 20 ng par million de cellules et par jour.

18. Lignée cellulaire pour utilisation selon la revendication 17, dans laquelle le trouble ophtalmique est un glaucome, de préférence dans laquelle le trouble ophtalmique est un glaucome à angle ouvert.

19. Lignée cellulaire pour utilisation selon la revendication 18, dans laquelle la production de PGF2a diminue la pression intraoculaire, stabilise la pression intraoculaire, ou à la fois diminue et stabilise la pression intraoculaire chez le patient.

20. Dispositif selon la revendication 4, lequel dispositif est destiné à être implanté dans une région cible d'un hôte receveur, dans lequel la ou les cellule(s) ARPE-19 encapsulée(s) sécrète(nt) PGF2a au niveau de la région cible.

21. Lignée cellulaire selon la revendication 19, dans laquelle la région cible est choisie dans l'ensemble constitué par le cerveau, le ventricule, la moelle épinière, l'humeur aqueuse et l'humeur vitrée de l'oeil, et la chambre postérieure et la chambre antérieure de l'oeil, de préférence dans laquelle la région cible est choisie dans l'ensemble constitué par l'humeur aqueuse et l'humeur vitrée de l'oeil, et la chambre postérieure et la chambre antérieure de l'oeil.

22. Procédé pour produire le dispositif de culture cellulaire implantable de la revendication 4, comprenant
a) la modification génétique d'au moins une cellule PE-19 pour qu'elle exprime la séquence d'acide nucléique de la SEQ ID NO : 1 ; et
b) l'encapsulation desdites cellules PE-19 génétiquement modifiées à l'intérieur d'une membrane semi-perméable.
